(19) Europäisches Patentamt / European Patent Office / Office européen des brevets

(11) **EP 4 621 674 A1**

(12) **EUROPEAN PATENT APPLICATION**

(43) Date of publication:
24.09.2025 Bulletin 2025/39

(21) Application number: 25159512.0

(22) Date of filing: **21.02.2025**

(51) International Patent Classification (IPC):
$G06Q\ 10/04^{(2023.01)}$    $G01N\ 33/00^{(2006.01)}$
$G06F\ 30/20^{(2020.01)}$    $G06Q\ 10/30^{(2023.01)}$
$G06Q\ 50/04^{(2012.01)}$    $G16C\ 60/00^{(2019.01)}$

(52) Cooperative Patent Classification (CPC):
G06Q 10/04; G01N 33/00; G06F 30/20;
G06Q 10/30; G06Q 50/04; G16C 60/00

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **22.03.2024 GB 202404188**

(71) Applicant: **Gulsine Ltd**
**London SW20 0PP (GB)**

(72) Inventor: **Ozdemir, Nazli**
**London, SW20 0PP (GB)**

(74) Representative: **Mathys & Squire**
**The Shard**
**32 London Bridge Street**
**London SE1 9SG (GB)**

(54) **COMPUTER-IMPLEMENTED METHOD TO DEFINE PRODUCT LIFESPAN**

(57) The present disclosure relates to a computer-implemented method to define the lifespan of products. The computer-implemented method for use in calculating the lifespan of a product, wherein the product comprises a plurality of materials, the method comprising: obtaining parameters relating to the plurality of materials of the product; obtaining a $Q_{10}$ value of each of the plurality of materials of the product, based on the obtained parameters; determining an overall $Q_{10}$ value of the product, using a set of rules and the $Q_{10}$ values of the plurality of materials; and outputting an appropriate ageing temperature and the overall $Q_{10}$ value for an accelerated ageing test of the product to calculate the lifespan of the product.

200

Obtain parameters relating to the plurality of materials of the product (205)

↓

Obtain the Q10 value of each of the plurality of materials of the product, based on the obtained parameters (210)

↓

Determine an overall Q10 value of the product, using a set of rules and the Q10 values of the plurality of materials (215)

↓

Output an appropriate ageing temperature and the overall Q10 value for an accelerated ageing test of the product to calculate the lifespan of the product (220)

Figure 2

**Description**

Field of the invention

The present disclosure relates to a computer-implemented method for use in calculating the lifespan of a product, for identifying the ageing test conditions for accelerated ageing tests of the product, and for obtaining a $Q_{10}$ value for accelerated ageing tests of the product.

Background

In numerous sectors, spanning both research and development as well as production and use, there is a significant reliance on specialised products across diverse conditions. Products, for example polymer based devices or medical components, age under the influence of three main factors, time, temperature, environment and stress dependent. As products age, their properties change with time. As a result, device manufacturers need to understand and appreciate the ageing of products to define the lifespan of the devices and attain them a reliable shelf-life. In this regard, product ageing knowledge is very valuable.

To optimise cost efficiency and minimise waste, devices are commonly utilised for their full shelf-life until reaching a point of failure. To guarantee that products can realise their designated life cycle and shelf-life to the fullest extent before experiencing deterioration, comprehensive testing is conducted. This testing often includes identifying the overall rate of ageing of a product, replicating the anticipated usage environment of the product, and subjecting the product to elevated temperatures until detrimental mechanical or chemical decomposition is observed.

Material industries and testing laboratories use material testing and accelerated ageing tests to simulate the life cycle of a product with short-term performance tests at elevated temperatures to predict the long-term performance of the product. This typically involves several years of rigorous testing to gather the necessary data. To accurately identify the lifespan of these materials or devices, first the rate of ageing of a product is required. Determination of ageing factors affecting the ageing of a product is extensive and necessitates climatic chambers, laboratory technicians, test samples, and other resources, resulting in a significant consumption of energy and materials. Upon identifying the overall rate of ageing of the product, also known as the ageing factor or $Q_{10}$ value of the product, accelerated aging tests are required to simulate the entire life cycle of the product until the point of failure. Similar to the testing of aging factors, this process demands substantial amounts of energy and resources over an extended duration.

To reduce energy consumption, cost, and labour requirements across material testing industries the rate of ageing is commonly taken as 2 as based on the general guidance from ASTMD1980F and Arrhenius equation. This value represents material decomposition via oxidation and does not accurately represent the most detrimental decomposition process in the majority of materials. Although, the research requirements are diminished by assuming a $Q_{10}$ value of 2, the accuracy of material characterisation is reduced, and the material or device will most likely require over-ageing in subsequent accelerated ageing tests. This over-ageing may lead to device failure or product rejection and an underestimated lifespan and shelf-life of the product. Over-ageing disadvantageously creates detrimental product waste and consumes resources and power which could be negated with an accurate $Q_{10}$ value. In cases where a $Q_{10}$ value is determined manually in a laboratory, it is common for manufacturers to overlook multiple mechanisms which take place during ageing of the product due to the sheer multitude of internal, external, and inter-material decomposition mechanisms that a product faces in its life cycle. Lifespan calculations may resultingly be adequate but not optimal.

In addition to substantial resource consumption and suboptimal accuracy, the duration of over-aged accelerated ageing studies is repeatedly hindering the time taken for products to be released to the market. In the current research climate, manufacturers are continuously producing new products to aid with issues arising in a plethora of industries. For example, industries relating to aerospace, automotive, electronics, nuclear cables, biotech, food packaging, hygienic healthcare products, and biopharmaceutical and medical devices require accelerated ageing material testing to ensure the durability and reliability of products over time. Because accelerated ageing tests take several years of rigorous testing for a scientist, there is a delay in utilizing cutting-edge products or components promptly, leading to the implementation of innovative products with imprecise lifespans which may already be improved upon by products in research and development.

Summary of the invention

Aspects of the invention are as set out in the independent claims and optional features are set out in the dependent claims. Aspects of the invention may be provided in conjunction with each other and features of one aspect may be applied to other aspects.

Embodiments of the disclosure are directed towards a computer-implemented method to define the lifespan of a product and to identify the ageing test conditions for accelerated ageing tests of the product. Embodiments of the disclosure may advantageously address the aforementioned problems and provide a method which can reduce the cost, labour, and consumption of energy and resources in material testing and accelerated ageing tests while providing a faster, efficient, and sustainable way of gaining an accurate lifespan and shelf-life of products.

A first aspect of the disclosure provides a computer-implemented method for use in calculating the lifespan of a product, wherein the product comprises a plurality of materials, the method comprising: obtaining parameters relating to the plurality of materials of the product; obtaining a $Q_{10}$ value of each of the plurality of materials of the product, based on the obtained parameters; determining an overall $Q_{10}$ value of the product, using a set of rules and the $Q_{10}$ values of the plurality of materials; and outputting an appropriate ageing temperature and the overall $Q_{10}$ value for an accelerated ageing test of the product to calculate the lifespan of the product.

Advantageously, such computer-implemented method for calculating the lifespan of a product may overcome the most difficult aspects of product development, determining an accurate $Q_{10}$ value and lifespan of a product. Typically, manufacturers bypass extensive material testing due to the abundant amount of experimental equipment, experimental space, and test samples needed to undertake such experiments. As a result, the reliability of the $Q_{10}$ value of a product may not be maximised and the accelerated ageing test and obtained lifespan of a product may be inaccurate. Obtaining a $Q_{10}$ value of each of the plurality of materials of a product and using these values to subsequently determine an overall $Q_{10}$ value of the product may advantageously provide a reliable $Q_{10}$ value for use in accelerated ageing tests and lifespan determination.

Further advantageously, the computer-implemented method may reduce the time taken to get a product to market. By outputting an accurate overall $Q_{10}$ value, the appropriate ageing temperature and duration of accelerated ageing test of the product can also be determined accurately using the Arrhenius equation. By way of comparison, conventional lifespan determining methods often provide inaccurate, typically lower, $Q_{10}$ values which require longer accelerated ageing tests. By negating the need for excessive accelerated ageing tests, the computer-implemented method may allow products to be released from research and development, for use in industries, quicker than other accelerated ageing tests and lifespan identification methods. The computer-implemented method, using its accurate $Q_{10}$ evaluation technique, may reduce the duration of accelerated ageing studies for products by up to six-fold. Reduction of the time taken for products to pass research and development may allow manufacturers to improve devices at a much greater rate, benefiting manufacturers, industries, and users.

The parameters relating to the plurality of materials may comprise at least one of factors relating to the environment, the polymer intrinsic properties of the plurality of materials, and the structure of the product. Advantageously, a large plethora of information about the product may highlight the most detrimental ageing mechanisms present in the materials of the product prior to any material testing. Narrowing down the scope of ageing mechanisms to be assessed for each of the plurality of materials reduces the experimental techniques required to accurately understand the chemical or mechanical processes which may actively deteriorate the materials of the product throughout its life cycle. Products released to the market through this computer-implemented method may contain valid integrity and robustness for long term use otherwise not witnessed in conventional testing methods.

In examples, the polymer intrinsic properties may comprise chemical formula, molecular weight, crosslinking mechanisms, chain structures, and processing methods.

The factors relating to the environment may comprise humidity, temperature, and pressure, and wherein these parameters are provided for the environments in which the product is for example assembled, intended to be stored, and intended to be used. Advantageously, understanding the different environmental factors that the product faces in different parts of its unique life cycle may allow the computer-implemented method to identify stages of the life cycle of the product and calculate the shelf-life or duration in which the product may be intended for use.

The structure of the product may comprise the product user required specification, information about the interfaces between the plurality of materials, and the joining mechanisms between materials. Modelling the kinetics of material deterioration is complex so specific information about the interactions between the materials in the product may advantageously allow for deterioration processes to be identified that may otherwise be missed or overlooked when manufacturers conventionally estimate the ageing mechanism for a given product.

In examples, each of the plurality of materials of the product are assessed for environmental stress cracking (ESC) prior to $Q_{10}$ evaluation.

In examples, the parameters relating to the plurality of materials may be provided for the whole of the lifecycle of the product, and wherein determining an overall $Q_{10}$ value of the product comprises determining an overall $Q_{10}$ value of the product for the whole lifecycle of the product. Advantageously, determining a $Q_{10}$ value of the product for the whole lifecycle of the product may allow the lifespan of the product to be reliably calculated for the whole lifecycle. This may be especially useful in industries such as medical devices or nuclear power plants, for example in pacemakers or reactors, where the life of a patients or operators are at risk.

In examples, the parameters of the plurality of materials may be provided for different portions of the lifecycle of the product, wherein determining an overall $Q_{10}$ value of the product comprises determining an overall $Q_{10}$ value of the product for a selected portion of the lifecycle of the product. Advantageously, determining a $Q_{10}$ value of the product for selected parts of the lifecycle of the product may allow the lifespan of the product to be calculated for each part of the lifecycle. The ageing mechanisms faced in different parts of the life cycle, for example assembly, storage, and transport, may be accurately predicted. This may be especially useful for understanding where high risk deterioration mechanisms occur in the production line or how different environments, such as unregulated storage housing or hotter usage climates, may affect the predicted life span of the products throughout the whole cycle. Further advantageously, manufacturers may use this information to alter assembly, storage, and transport conditions to reduce parts of the life cycle with fast ageing mechanisms and to extend the shelf-life of their product.

Obtaining a $Q_{10}$ value of each of the plurality of materials of the product may comprise performing a lookup in a database of $Q_{10}$ values. Advantageously, performing a lookup in a database may identify if the plurality of materials of the product have been previously investigated for their accurate and unique $Q_{10}$ ageing value. The previously determined accelerated ageing test conditions and $Q_{10}$ value of the product may be outputted at a rapid rate with confidence and without laboratory testing. The disclosed computer-implemented method may, therefore, advantageously reduce the experimental requirements needed to predict the long-term performance of the product by obtaining $Q_{10}$ values of a product from previous material analysis, without the need for frequent material testing in a laboratory. This may be advantageous compared to traditional $Q_{10}$ value evaluation experiments which require an abundant amount of experimental equipment, experimental space, and test samples to undertake such experiments. Reduction of experimental techniques undertaken on the product may further reduce the time and cost taken for the lifespan of the product to be calculated.

Performing a lookup in a database of $Q_{10}$ values may comprise selecting a $Q_{10}$ value of a material in the database that is similar to a material in the product, wherein the material is similar to a material in the database of $Q_{10}$ values if the parameters of the material are within a selected threshold level of similarity. In examples, the selected threshold level of similarity comprises an 98.5% overlap of parameters wherein the overlap may be, for example, an 98.5% overlap in material composition. Advantageously, the use of a select threshold of similarity of the parameters of the material of the product to a material in the database allows an accurate $Q_{10}$ value for the material to be picked from the large dataset of $Q_{10}$ values, even if the exact material has not been manually assessed previously. Correlations between types of materials, the environmental factors, and the ageing characteristics may be established and the need for manual testing of materials in a product further diminished.

Obtaining parameters of the material of the product may comprise performing a lookup in a database of parameters for materials in products, wherein the lookup is performed based on part names and classification and identifies a material that is similar to a material in the product, wherein the material is similar to a material in the database if the parameters of the material in the database and parameters of the material in the product are within a selected threshold level of similarity. Advantageously, performing a lookup based on part names and classification may provide a precise and structured way to retrieve information about the materials in the database making it efficient to find specific parameters and data of materials of interest. This approach may streamline the process of matching the materials of the product to materials in the database without the need for laboratory space or analysis from scientists. Further advantageously, using a select threshold level of similarity between parameters of the materials of the product and of the database may allow proximal materials suitable for the same lifecycle to be chosen. This may be especially useful for new iterations of previous products which comprise a minute difference in composition or structural design but comprise similar parameters.

In examples, the computer-implemented method may comprise an internal database and an external database. In further examples, the computer-implemented method may comprise an internal database and interact with a plurality of external databases.

The classifications for the lookup in a database may include the intended use of the product, the factors relating to the environment, and the polymer intrinsic properties of the plurality of materials. Advantageously, performing a lookup using multiple classifications allows numerous materials of the database to be chosen efficiently and put through the selective threshold test to find the most appropriate material in the database and the most reliable $Q_{10}$ value.

In examples, a material may be similar to a material in the database if the materials have the same polymer intrinsic properties. Advantageously, identifying similar materials by their polymer intrinsic properties may identify the common ageing mechanisms, or highest risk and fastest deterioration processes, of those similar products in the same environment.

In examples, materials of a product may be similar to materials of a product in the database if the product faces the same environments when it is assembled, stored, and used. Advantageously, identifying similar materials by their environment may identify the ageing mechanisms with the highest risk or fastest deterioration process in multiple products at the same severe or intense environments in, for example, assembly, storage, transport, or use.

The $Q_{10}$ value of a material may be obtained manually. Advantageously, if a similar material is not identified for a material in the product by the lookup in a database, then the $Q_{10}$ value of the material may be obtained manually through experiment, without necessitating the whole product to be tested. The overall $Q_{10}$ of the product may still accurately be determined without traditional material testing or carbon-intensive over-ageing accelerated ageing tests.

In examples, the $Q_{10}$ value of a material is obtained using a dynamic mechanical analyser (DMA). Advantageously, the DMA collects material data in roughly a day which is a lot quicker than conventional testing, for example mechanical testing, which collects data after years of testing. In further examples, the $Q_{10}$ value of a material is obtained using differential scanning calorimetry (DSC). Advantageously, DSC provides data about multiple products if only one $Q_{10}$ value of a product is known which reduces the need for mechanical or DMA testing and a lookup in a database of $Q_{10}$ values.

The set of rules, which are used to determine the overall $Q_{10}$ value of the product, may disregard any $Q_{10}$ values of 2. $Q_{10}$ values of 2 are associated with oxidation and the ageing mechanisms of semicrystalline polymer regions. These ageing mechanisms occur a lot slower than those associated with amorphous polymer regions. Advantageously, disregarding these lower risk and rate ageing mechanisms helps identify the ageing mechanism and $Q_{10}$ value which poses the greatest risk to a product.

The step of determining an overall $Q_{10}$ value of the product, using a set of rules and the $Q_{10}$ values of the plurality of materials may comprise selecting an overall $Q_{10}$ value of the product using the set of rules, from the plurality of $Q_{10}$ values of the materials of the product, as the highest $Q_{10}$ value if the spread of $Q_{10}$ values is less than a selected threshold. Advantageously, determining the overall $Q_{10}$ value to be the highest $Q_{10}$ value identifies the ageing mechanism which occurs at the fastest rate. As a result, the appropriate ageing temperature and accelerated ageing test duration, using the Arrhenius equation, that is outputted may provide the fastest accelerated ageing test results with confidence and reduced power consumption.

In examples, the step of determining an overall $Q_{10}$ value of the product, using a set of rules and the $Q_{10}$ values of the plurality of materials may comprise selecting an overall $Q_{10}$ value of the product using the set of rules, from the plurality of $Q_{10}$ values of the materials of the product, as the median $Q_{10}$ value if the spread of $Q_{10}$ values is greater than a selected threshold. Advantageously, determining the overall $Q_{10}$ value to be the median $Q_{10}$ value if the spread of values is large may take into account the effect of ageing mechanisms which are apparent but small. Purely for illustrative purposes, the set of rules may acknowledge that a slow ageing mechanism provided by a large portion of the product needs to be accounted for, rather than placing all significance on a minute part of the product with a very fast rate of ageing. For example, a product intended to be used under water and to never encounter excessive load may witness the fastest ageing mechanism of tensile stress but will experience more dominant deterioration or ageing through hydrolysis. In this case, the $Q_{10}$ factor of hydrolysis cannot be overlooked by the high $Q_{10}$ factor of tensile stress.

The step of determining an overall $Q_{10}$ value of the product, using a set of rules and the $Q_{10}$ values of the plurality of materials may comprise defining a weighting of the $Q_{10}$ values of each of the plurality of materials using the set of rules. Advantageously, defining weightings of the $Q_{10}$ values of the plurality of materials using a set of rules may provide a reliable overall $Q_{10}$ value of the product which is not influenced unfairly by the fastest or slowest acting ageing mechanisms. Accurate $Q_{10}$ factors, determined by methods as such, may instil confidence in the market and users, such as in medical practices or food packing, where the lifespan of products is a cause of health concerns.

The set of rules may be based on at least one of: (i) historic ageing test data and failed test data; (ii) the intended use of the product and the most prominent chemical or mechanical processes on the product; and (iii) the duration and/or characteristics of different parts of the lifecycle of the product. Advantageously, historic ageing test data and failed test data may teach the set of rules about which combinations of $Q_{10}$ values have produced an inaccurate overall $Q_{10}$ value of the product; the inaccurate overall $Q_{10}$ value is consequently not reassigned to a similar product. Moreover, historic ageing test data may allow the set of rules to rapidly identify the overall $Q_{10}$ value of a product that has been seen before, without obtaining a $Q_{10}$ value of each of the plurality of materials of the product. Further advantageously, the intended use of the product and the most prominent chemical or mechanical processes of the product may teach the set of rules which ageing mechanisms require more or less weighting. Additionally, basing the rules off the duration and/or characteristics of different parts of the lifecycle of the product advantageously may allow the computer-implemented method to ignore $Q_{10}$ values which do not act for a substantial amount of time or cause any significant effect on the product.

The appropriate ageing temperature may be obtained by the Arrhenius equation and the $Q_{10}$ value, ambient temperature, desirable lifespan, and accelerated ageing time of the product. In examples, the accelerated ageing time is obtained by the Arrhenius equation and the $Q_{10}$ value, ambient temperature, desirable lifespan, and accelerated ageing temperature of the product. Advantageously, the choice of appropriate ageing temperature or accelerated ageing time allows accelerated ageing tests to be altered to suit the laboratory in use and the needs of the manufacturer.

The outputted appropriate ageing temperature and the overall $Q_{10}$ value for an accelerated ageing test of the product may be inputted into the memory of the database for use in further calculations of the lifespan of products. Advantageously, inputting the outputted appropriate ageing temperature and overall $Q_{10}$ value for the accelerated ageing test of each product back into the database may reduce the need for future manual material $Q_{10}$ value experiments and the time it takes for the lifespan of products to be calculated. Over multiple iterations, the computer-implemented method may generate a correlation between parameters of the product and ageing characteristics whereby inputting the parameters of the product into the method promptly outputs appropriate ageing test conditions and a $Q_{10}$ value. In examples, this correlation may also output the lifespan prediction of a product based off previous product accelerated ageing test results. Further advantageously, previously defined lifespans may be revised to extend the time in use of products. The circular nature of product development and testing via the computer-implemented method may support product life cycles and create a circular economy. Validation of products may be simplified and products will be released to market faster with more confidence and with extended shelf-life.

Another aspect of the disclosure provides a computer-implemented method for identifying the ageing test conditions for accelerated ageing tests of a product, wherein the method comprises: obtaining at least one parameter of the product; performing a lookup in a database, wherein the database comprises historic ageing test data of previous products; identifying at least one previous product in the database which comprises at least one common parameter of the product; obtaining weighted $Q_{10}$ values of previous products with at least one parameter in common with the product using a set of rules; and outputting the ageing test conditions of the previous product with the highest weighted $Q_{10}$ value.

Advantageously, the disclosed computer-implemented method may reduce the experimental requirements needed to predict the long-term performance of a product by obtaining $Q_{10}$ values of a product from previous material analysis, without the need for frequent material testing in a laboratory. This may be advantageous compared to traditional rate of ageing, or $Q_{10}$, experiments which require an abundant amount of experimental equipment, experimental space, and test samples to undertake such experiments.

Advantageously, identifying the ageing test conditions for accelerated ageing tests of a product by a computer-implemented method, parameter information, database lookup, and a set of rules may provide accurate time efficient appropriate ageing test conditions and accurate lifespan information of a product rapidly. The time of research and development of a product may be significantly reduced, accelerating the time to market release. The reduction in time to market release and the increase in reliability of released products may advantageously boost the share prices and sales per annum of manufacturing companies. The economies and industries of product development in turn may increase in productivity and research output.

The identified ageing test conditions may comprise an appropriate ageing temperature, $Q_{10}$ value, desirable lifespan, and accelerated ageing time of the product.

The historic ageing test data within the database may comprise the parameters, ageing temperature, $Q_{10}$ value, lifespan, and accelerated ageing time of the product. Advantageously, this plethora of information will allow products with similar or the same parameters to obtain ageing test data and optionally a lifespan without the need for repeated material testing and accelerated ageing tests.

In examples, the at least one parameter of the product may comprise factors relating to the environment, the polymer intrinsic properties of the product, and the structure of the product. Advantageously, encompassing parameters of a product relating to external events and internal ageing mechanisms provides a $Q_{10}$ value and appropriate ageing test conditions that replicate the true lifecycle of the product when in use rather than at room temperature as seen in conventional ageing tests.

In examples, the parameters relating to the environment may comprise humidity, temperature, and pressure, and wherein these parameters are provided for the environments in which the product is assembled, intended to be stored, and intended to be used. Advantageously, the computer-implemented method may identify the separate parts of the life cycle of the product and may quickly recalculate the lifespan and shelf-life of the product if a part of the lifecycle is altered slightly. This is an advantage as the product, or future similar products, may not require extensive re-experimentation when minute changes are made to parts of its life cycle such as the assembly, storage, or use. Further advantageously, products may face different storage, transport, and usage conditions around the globe, and understanding these environments may allow for an accurate life span and shelf-life calculation of all products produced and used around the world.

In examples, the parameters relating to the structure of the product may comprise the product user required specification, information about the interfaces between the plurality of materials in the product, and joining mechanisms between the plurality of materials in the product. Advantageously, the provided parameters allows the material testing to focus on the $Q_{10}$ value of the property that intends a greater risk to long term product integrity.

The step of obtaining weighted $Q_{10}$ values may comprise the step of using a set of rules to add weighting to obtained $Q_{10}$ values of previous products with at least one parameter in common with the product.

The set of rules may be based on at least one of: (i) historic ageing test data and failed test data; (ii) the intended use of the product and the most prominent chemical or mechanical processes on the product; and (iii) the duration and/or environmental conditions of different parts of the lifecycle of the product.

The outputted ageing test conditions and parameters of the product may be added to the database for use in further identification of ageing test conditions for accelerated ageing tests of products.

Another aspect of the disclosure provides a computer-implemented method for identifying the ageing test conditions for accelerated ageing tests of a product, wherein the method comprises: obtaining parameters relating to the product; determining the parameters of the product based on a lookup of previous products stored in a database; identifying previous products with at least one parameter in common with the product; determining a $Q_{10}$ value for the product based on the $Q_{10}$ value of a previous product in the database; and outputting the $Q_{10}$ value for the product and an appropriate ageing temperature for use in accelerated ageing tests of the product.

Advantageously, identifying and obtaining conditions for accurate lifespan predictions may globally diminish unnecessary premature product waste, allow for products to be re-used, and reduce the carbon-foot print of industries.

The parameters of the product may comprise at least one of factors relating to the environment, the polymer intrinsic properties of the plurality of materials, and the structure of the product.

The factors relating to the environment may comprise humidity, temperature, and pressure, and wherein these parameters are provided for the environments in which the product is assembled, intended to be stored, and intended to be used.

The structure of the product may comprise the product user required specification, information about the interfaces between the plurality of materials, and the joining mechanisms between materials.

The selective threshold may be a 98.5% overlap of parameters in the product and previous products. In examples, the selective threshold may be a 98.5% overlap in composition of two comparative materials. In further examples, the selective threshold may be a 98.5% overlap in the environments witnessed by the two comparative materials in their life cycles.

In examples, the product may comprise only one material. In further examples, the product may comprise any number of materials.

The ageing test conditions may be outputted for use in determining the lifespan of the product.

The ageing test conditions of the product may be fed back to the database for use in further identifying the ageing test conditions for accelerated ageing tests of products.

Another aspect of the disclosure provides a computer-implemented method for obtaining a $Q_{10}$ value for accelerated ageing tests of a product, wherein the method comprises: performing a time temperature superposition technique on aged samples of the product; obtaining material characteristic values for each of the aged samples of the product for each ageing duration; iteratively determining a $Q_{10}$ value for the product that provides a closest coefficient of determination to 1; outputting the determined $Q_{10}$ value for the product.

Advantageously, determining a $Q_{10}$ value for the product by closest coefficient of determination to 1 outputs a $Q_{10}$ value of high reliability without excessive mathematical manipulation of data.

Another aspect of the disclosure provides a computer-implemented method for obtaining a $Q_{10}$ value for accelerated ageing tests of a product, wherein the method comprises; obtaining at least one $Q_{10}$ value of a first product; performing differential scanning calorimetry on the first product; determining the ratio of semicrystalline chain structure to amorphous chain structure present in the product, using the differential scanning calorimeter analysis data of the first product; determining the ratio of semicrystalline chain structure to amorphous chain structure present in a second product using differential scanning calorimetry; predicting the $Q_{10}$ value of a second material using (i) the $Q_{10}$ value of the first product, (ii) the determined ratio of semicrystalline chain structure to amorphous chain structure present in the first product, and (iii) the determined ratio of semicrystalline chain structure to amorphous chain structure present in the second product.

Advantageously, obtaining multiple $Q_{10}$ values of a product, or $Q_{10}$ values of other similar products, by way of one manually determined $Q_{10}$ value and the ratio of semicrystalline chain structure to amorphous chain structure reduces the time taken to determine the overall $Q_{10}$ value of a product and get the product to market.

Another aspect of the disclosure provides a computer-implemented method for use in calculating the lifespan of a skin sample, the method comprising: obtaining parameters relating to the skin sample; obtaining $Q_{10}$ values of the skin sample, based on the obtained parameters; determining an overall $Q_{10}$ value of the skin sample, using a set of rules and the obtained $Q_{10}$ values; and outputting an appropriate ageing temperature and the overall $Q_{10}$ value for an accelerated ageing test of the skin sample to calculate the lifespan of the skin sample.

Drawings

Embodiments of the disclosure will now be described, by way of example only, with reference to the accompanying drawings, in which:

Figure 1 shows a flow chart showing an example method for calculating the lifespan of a product which provides context for how the example computer-implemented methods of Figures 2, 3, 7, 8, 9, and 10 are utilised;

Figure 2 shows a flow chart of a first example computer-implemented method of the present disclosure which could be used in the example method of Figure 1;

Figure 3 shows a more detailed flow chart of the first example computer-implemented method that is depicted in Figure 2 with additional embodiments;

Figure 4A shows a flow chart of an example method followed to determine the $Q_{10}$ value of a product manually;

Figure 4B shows example averaged tensile strength values obtained from samples after varied ageing times;

Figure 5 shows a flow chart of an example procedure taken to manually determine the overall $Q_{10}$ value of a product using the $Q_{10}$ values of the plurality of materials of the product;

Figures 6A and 6B show flow charts of worked examples of the procedure taken in Figure 5 to determine the overall $Q_{10}$ value of a product;

Figure 7 shows a flow chart of a second example computer-implemented method of the present disclosure which could be used in the example method of Figure 1;

Figure 8 shows a more detailed flow chart of the second example computer-implemented method that is depicted in Figure 7 with additional embodiments;

Figure 9 shows a flow chart of the third example computer-implemented method of the present disclosure which could be used in the example method of Figure 1;

Figure 10 shows a more detailed flow chart of the second example computer-implemented method that is depicted in Figure 9 with additional embodiments;

Figure 11 shows a first example system configured to perform the computer-implemented method of the present invention, for example configured for use with the method of Figure 2;

Figure 12 shows a second example system configured to perform the computer-implemented method of the present invention, for example for use with the computer-implemented method of Figure 10;

Figure 13 shows a graph depicting the life cycle of an example product which may be analysed by the computer-implemented methods of Figures 2, 3, 7, 8, 9, and 10;

Figure 14A shows a graph depicting the creep curves of two samples of a product, aged at different temperatures under a 32 MPa load, obtained through DMA;

Figure 14B shows a graph of the high temperature time temperature superposition (TTS) master curve of the two samples depicted in Figure 14A;

Figure 15A shows a first example TTS creep master curve obtained for a product;

Figure 15B shows a second example TTS creep master curve obtained for a product.

<u>Specific description</u>

Embodiments of the claims relate to a computer-implemented method for use in calculating the lifespan of a product, for identifying the ageing test conditions for accelerated ageing tests of the product, and for obtaining a $Q_{10}$ value for accelerate ageing tests of a product. In particular, the present disclosure relates to an efficient and streamlined method utilising parameters of the product, a database of $Q_{10}$ values and historic ageing test data of previous products, a set of rules, and a lookup mechanism, wherein the set of rules identify the ageing mechanism with the highest risk to product integrity and appropriate accelerated ageing test conditions for a product.

The steps and processing of information illustrated in the figures, including, but not limited to, any system, block, and flow diagrams, may typically be performed in the same or in a different serial or parallel ordering and/or by different components and/or processes, etc., and be combined with other steps in other examples and figures, unless this disables the embodiment or a sequence is explicitly or implicitly required (e.g., for determining the $Q_{10}$ value of the product, using a set of rules and the $Q_{10}$ values of the plurality of materials, the $Q_{10}$ values of the plurality of materials must be obtained prior to determining, although the set of rules may already be ordering the importance of $Q_{10}$ values already obtained, while manual testing of one material is taking place).

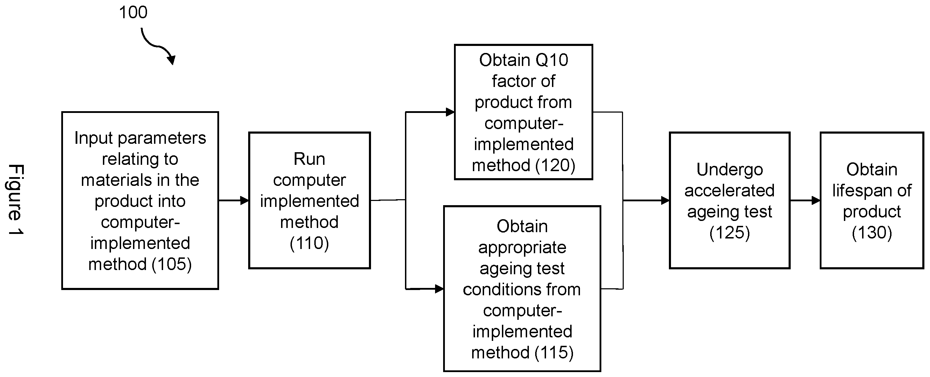

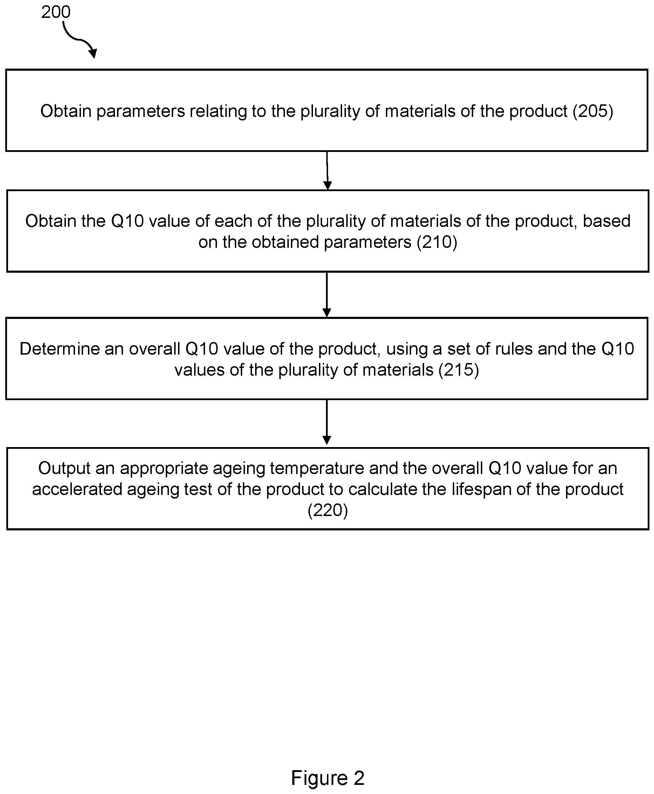

An example method for calculating the lifespan of a product, and providing context for how the computer-implemented methods of the present disclosure are applied, is shown in Figure 1. The computer-implemented methods which Figure 1 provides context for are shown in Figures 2, 3, 7, 8, 9, and 10.

Firstly, the method 100 comprises inputting parameters relating to the plurality of materials of a product 105 into a computer-implemented method 110 which is used in calculating the lifespan and appropriate ageing test conditions of the product. In the example methods provided herein, a user inputs the parameters 105 into the computer-implemented method 110 wherein the parameters 105 relate to characteristics of the product or plurality of materials of the product. However, the skilled person will understand that other parameters relating to materials or products may be inputted into the computer-implemented methods 110 of this disclosure. The parameters inputted into the computer-implemented method shall be discussed in more depth with reference to Figure 2.

The method 100 also comprises running the computer-implemented method used for calculating the lifespan of a product 110 and obtaining, from the computer-implemented method 110 used in calculating the lifespan of a product, a $Q_{10}$ value 120 and appropriate ageing test conditions 115 of the product as defined by the inputted parameters 105. In examples, the appropriate ageing test conditions outputted by the computer-implemented methods 110 optionally comprise an appropriate accelerated ageing time or an appropriate ageing temperature depending on the desired conditions of the accelerated ageing tests 125 and the secondary ageing mechanism that may occur in the product. For example, as determined by the Arrhenius equation, a low ageing temperature will require a longer accelerated ageing test 125, whereas a high ageing temperature will require a shorter accelerated ageing test 125. The optional choice of appropriate ageing test conditions allows a manufacturer to decide whether they require or prefer a shorter test duration or colder test environments. The accelerated ageing test 125 of method 100 then comprises identifying the corresponding accelerated ageing time or accelerated ageing temperature by way of the Arrhenius equation,

$$t_{AA} = \frac{t_{DR}}{Q_{10}^{\frac{T_{AA}-T_{RT}}{10}}}.$$

In this equation $t_{AA}$ and $t_{DR}$ are the accelerated ageing and desired real times, respectively, and $T_{AA}$ and $T_{RT}$ are the accelerated ageing and real-world temperatures. In other examples, the appropriate ageing temperature is chosen as the highest temperature that the product can face before secondary ageing mechanisms occur.

The method 100 then comprises undergoing an accelerated ageing test 125 of the product, using the obtained $Q_{10}$ factor 120 and appropriate ageing test conditions 115, and obtaining a lifespan of the product 130 from the accelerated ageing test 125. The accelerated ageing test 125 optionally comprises storing the product at a given temperature for a given duration and analysing the changes in the properties of the product to predict its long-term performance and durability. The obtained lifespan of the product comprises a numeric value that defines the period during which the product remains functional or usable for its intended purpose. The lifespan, or period in which the product is usable before exhibiting product failure, is the time in which over 50% of the material property is unaffected. Once the material exhibitions over a 50% loss in property, the lifespan has ended.

The method 100 of Figure 1 is configured to provide the computer-implemented method 110 with the means to determine the $Q_{10}$ values 120 and appropriate ageing test conditions 115 of a product, wherein the means for doing so is the plethora of parameters inputted to the computer-implemented method 110. The method is then configured to use said $Q_{10}$ values 120 and appropriate ageing test conditions 115 for calculating the life span of the products 130 by way of an accelerated ageing test 125. In use, this method 100 allows a user to quickly and efficiently obtain the lifespan of the product 130 of interest, given they have information about the product 105.

Figure 2 shows a flow chart of a first example computer-implemented method 200 of the present disclosure which could be used in the example method 100 of Figure 1 for obtaining a $Q_{10}$ value 120 and appropriate ageing test conditions 115 and for use in calculating the lifespan of a product. In this example, the computer-implemented method 200 comprises obtaining parameters relating to the plurality of materials of the product 205, obtaining the $Q_{10}$ value of each of the plurality of materials of the product, based on the obtained parameters 210, determining an overall $Q_{10}$ value of the product, using a set of rules and the $Q_{10}$ values of the plurality of materials 215, and outputting an appropriate ageing temperature and the overall $Q_{10}$ value for an accelerated ageing test of the product to calculate the lifespan of the product 220.

As discussed, with reference to Figure 1, the parameters obtained in step 205 by the computer-implemented method 200, or any other computer-implemented method of this disclosure such as 300, 700, 800, 900, and 1000, relate to characteristics of the product or plurality of materials of the product. In examples, obtaining parameters comprises a user inputting data by, for example, a user collection interface. In other examples obtaining parameters of the product comprises interacting with external databases by way of, for example, a data collection module, wherein the external databases may have been sourced by experimental equipment or experimental testing.

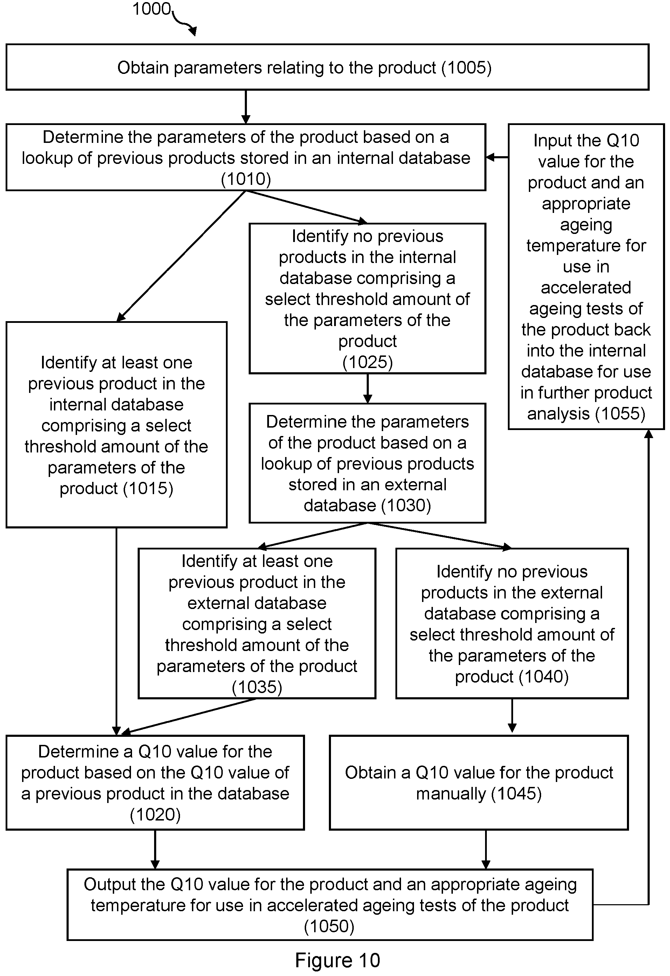

The characteristics of the product optionally comprise the User Requirement Specification (URS) and polymer intrinsic structure of the product. The URS optionally comprises information about the intended life cycle and environment of the product, wherein the life cycle comprises, for example, assembly, sterilisation, shipping, transport, storage, and use, and wherein the environment of the product is optionally defined for each part of the life cycle and includes, for example, humidity, temperature, and pressure values. Details about the intended life cycle and environment of products is described in greater depth with reference to Figure 10, which depicts an example product life cycle.

The polymer intrinsic structure of a product optionally comprises information about the structure of the product, such as the interfaces between the plurality of materials and the joining mechanisms between materials, the chemical formula of the materials, molecular weight of materials, crosslinking mechanisms, chain structures, and processing methods of the materials. However, the skilled person will understand that other characteristics relating to materials or products 105 may be inputted into the computer-implemented methods of this disclosure, for example unintended material changes over time such as the appearance of crystallinity changes, cracking, crazing, discolouration, swelling, shrinkage, and composition changes.

Furthermore, the computer-implemented method 200 may optionally comprise outputting an appropriate accelerated ageing time at step 220 instead of an appropriate ageing temperature depending on the desirable conditions of the accelerated ageing test of the product as discussed with reference to Figure 1. The appropriate ageing temperature outputted by the computer-implemented method 200 at step 220, and all other example computer-implemented methods of the disclosure, optionally depends on the parameters of the plurality of materials of the product. The appropriate ageing temperature is defined as the highest temperature with which the plurality of materials of the product can face before secondary unwanted ageing mechanisms occur. In examples, thermal degradation may occur around 80 degrees Celsius so an appropriate ageing temperature may be chosen at 60 degrees Celsius to avoid thermal degradation affecting the plurality of materials of the product. The obtained parameters relating to the plurality of materials of the product in step 205 help define the appropriate ageing temperature chosen and, in examples, the set of rules also output an appropriate ageing temperature based off previous historic ageing data and environmental factors. The set of rules, and their influential factors, will be discussed in greater detail below.

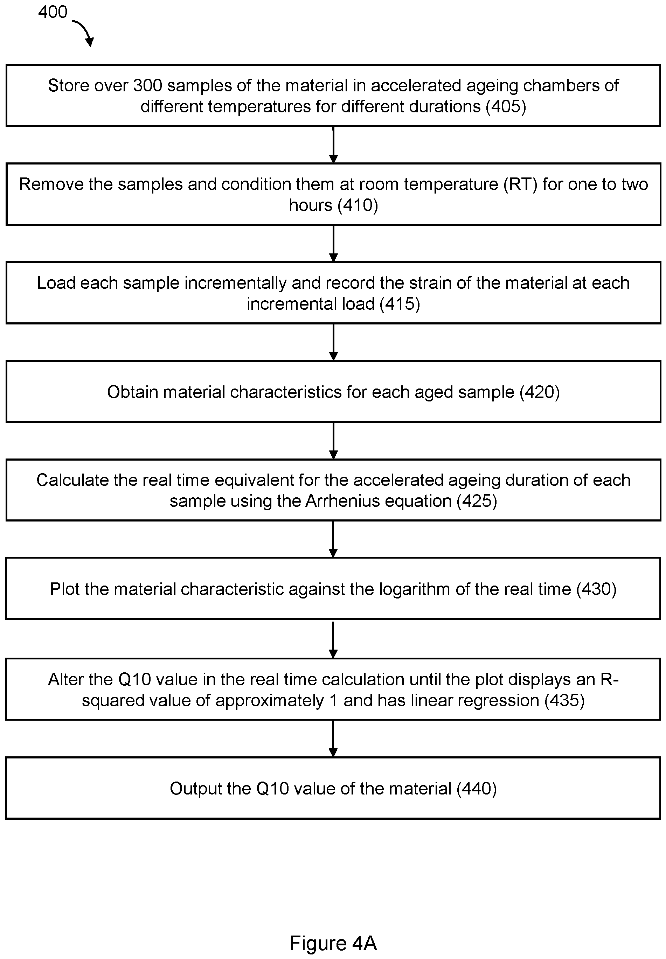

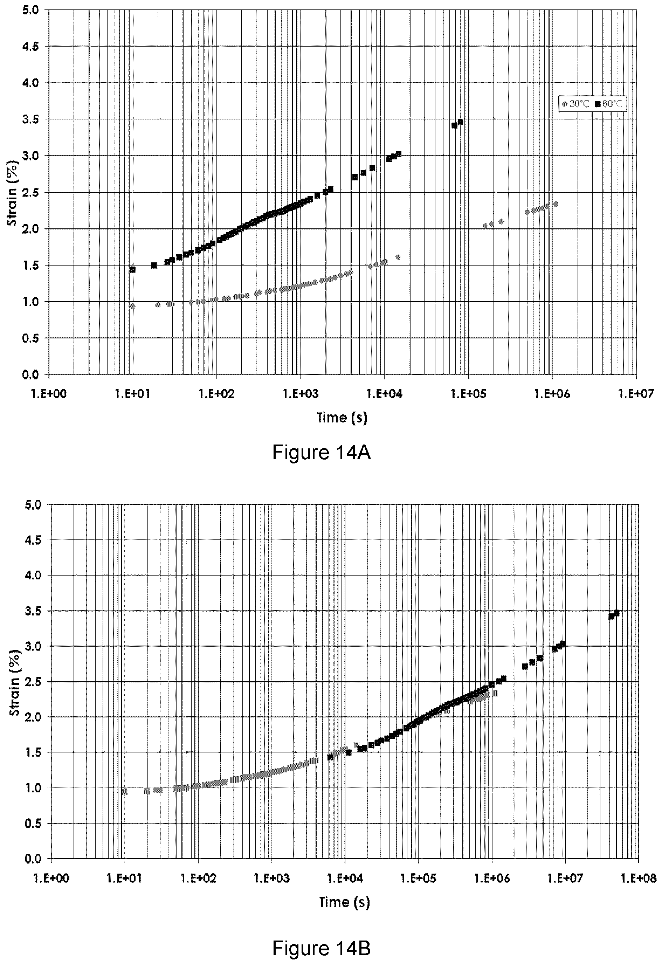

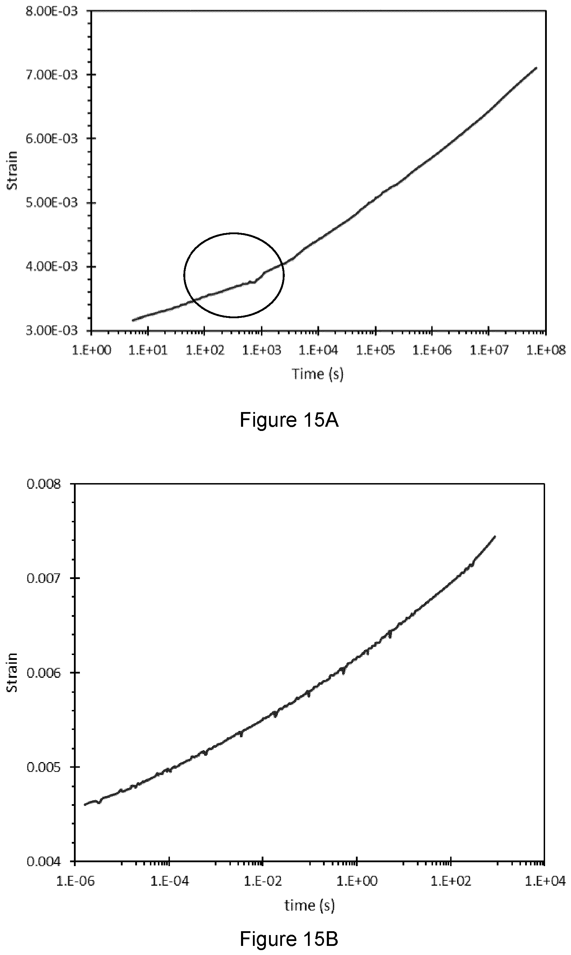

In examples, the $Q_{10}$ value of each of the plurality of materials of the product obtained at step 210 are obtained manually through experimental material testing, wherein the obtained parameters relating to the plurality of materials of the product 205 are configured to provide information about the ageing mechanisms occurring in each of the plurality of materials and information about which of these mechanisms may pose the greatest deterioration risk or have the fastest ageing rate on the integrity of each of the plurality of materials. In examples, possible ageing mechanisms that occur in a material are oxidation, hydrolysis, stress relaxation, cyclic fatigue, diffusion of volatiles, and environment stress cracking. Each manual test is configured to quantify so-called physical aging mechanisms occurring in each of the plurality of materials into a $Q_{10}$ value by exposing the sample to a series of isothermal ageing steps, collecting data at each step and collating said data with the Arrhenius equation and a TTS method. Manual testing can be done with mechanical testing equipment, more preferably with DMA, or most preferably by differential scanning calorimetry (DSC). An example manual test is depicted in Figure 4A and further examples are discussed with reference to Figures 14A, 14B, 15A, and 15B.

The computer-implemented method 200 optionally uses a set of rules, discussed further with reference to step 215, to identify the ageing mechanisms occurring in each of the plurality of materials which pose the greatest deterioration risk or have the fastest ageing rate on the integrity of each of the plurality of material. The ageing mechanisms acting on each of the plurality of materials with the highest risk are identified and tested experimentally, whereby the overall $Q_{10}$ value of each of the plurality of materials of the product are obtained 210 from the experiment. The method in which the $Q_{10}$ value of each material is obtained 210 manually is discussed in greater detail with reference to the example illustrated in Figure 4A and further ways of manually obtaining $Q_{10}$ values of a material or product are discussed with reference to Figures 14A, 14B, 15A, and 15B.

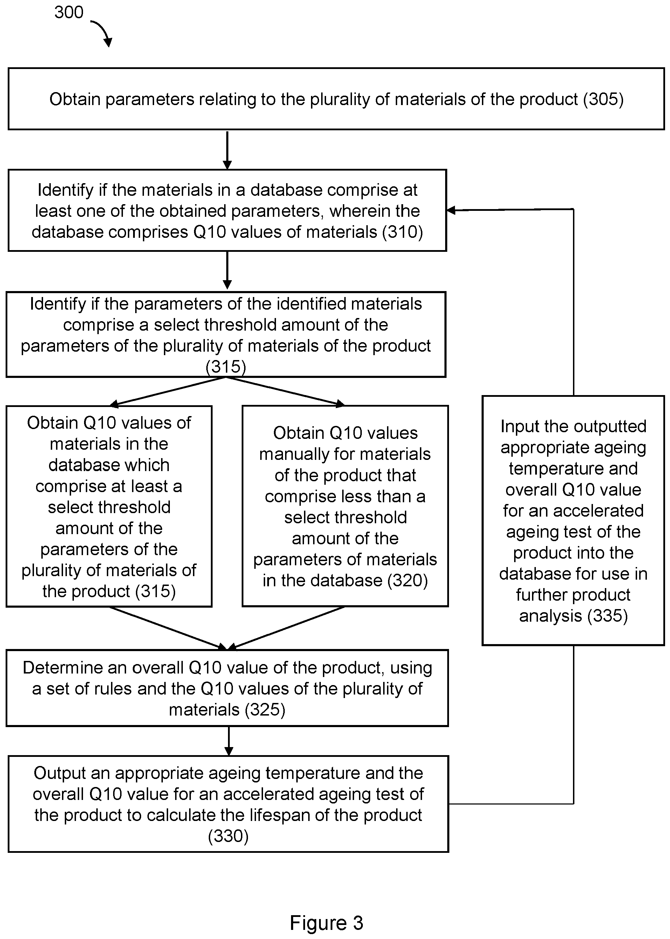

In examples where the $Q_{10}$ value of each of the plurality of materials of the product is obtained manually, the computer-implemented method may also comprise an additional two steps between 205 and 210 of storing material test data from each manual experiment and calculating the $Q_{10}$ value of each of the plurality of materials based on this stored data, using, for example, a mathematical platform. In other examples, like that of Figure 3, 7, 8, 9, and 10, the $Q_{10}$ value of each of the plurality of materials of the product are optionally obtained 210 from a database of $Q_{10}$ values, wherein the database optionally comprises $Q_{10}$ values, parameters, and historic ageing test data of previous materials or products. Databases which optionally provide $Q_{10}$ values of a material or product are discussed in greater detail with reference to Figures 3, 7, 8, 9, and 10.

In the example computer-implemented method 200 of Figure 2 and all further discussed computer-implemented method examples of Figures 3, 7, 8, 9, and 10, a portion of the set of rules used for determining an overall $Q_{10}$ value of the product in step 215 are optionally based on generic statistical reasoning, wherein, for example, if the spread of $Q_{10}$ values is small, the largest $Q_{10}$ value is chosen and indicates the fastest ageing mechanism which shall cause the most damage to the product but if the spread of $Q_{10}$ values is large, the median $Q_{10}$ value is chosen and indicates the dominant ageing mechanism occurring in the product. In other examples, where the data is mostly consistent but the spread is large, an average $Q_{10}$ value is chosen to accurately represent the ageing rate of the product.

The other portion of the set of rules in step 215 and the methods of Figures 3, 7, 8, 9, and 10 are optionally based, but not solely dependent, on historic ageing test data and failed test data, the intended use of the product and the most prominent chemical or mechanical processes on the product, and the duration and/or characteristics of different parts of the lifecycle of the product. These optional features are configured to instruct the set of rules on the importance of certain materials, environments, products, and ageing mechanism combinations and how they impact the overall ageing mechanism of the product. For example, for the purpose of illustration, historic ageing test data allows the set of rules to highlight if a similar spread of $Q_{10}$ values has occurred before for a previous product and to resultingly output the accurate overall $Q_{10}$ value of the previous product with confidence. Failed test data, on the other hand, can highlight when an overall $Q_{10}$ value was previously wrongly assigned to a product and inhibit the computer-implemented method from outputting a $Q_{10}$ value for a similar product. The intended use of a product and the most prominent processes acting on the product, identified using the obtained parameters in step 205, teaches the set of rules about which $Q_{10}$ values to place more weighting or importance upon. For example, a pacemaker will endure some form of oxidation in production, storage, and assembly but will also experience extreme hydrolysis, corrosion, or electronic component degradation from temperature and bodily fluids. Factors affecting the deterioration of the pacemaker in the body may form a larger weighting than those occurring at room temperature outside the body. The intended use in the human body signifies that hydrolysis may be the most prominent degradation process. As a result, the overall $Q_{10}$ value of the pacemaker should be centred around hydrolysis. Likewise, information about the lifecycle of the product also narrows down which acting $Q_{10}$ values are dominant on the product. These set of rules are configured to consider the ageing mechanisms of individual materials and product as a whole. For example, leaching, corrosion, and abrasion between materials would not be considered for the $Q_{10}$ values of each of the plurality of materials of a product but may be identified and quantified in the overall $Q_{10}$ value of the product. The skilled person will, however, understand that other statistical reasoning or historic data may be used to influence the decisions made by the set of rules in the computer-implemented methods of Figures 2, 3, 7, 8, 9, and 10.

Table 1 shows example $Q_{10}$ values, a, b, c, and d, of a medical plastic obtained through experimental testing. The overall $Q_{10}$ value of the plastic is chosen as c due to an example rule of thumb for medical plastics, other than composites, which defines that:

$$c > a > b \,\&\, d.$$

The skilled person will understand that many other material dependent rules known to scientists and experts will exist in the set of rules and cannot all be explained in the present disclosure.

| Ageing Mechanism | $Q_{10}$ Value | Testing mechanism |
|---|---|---|
| Physical | a | Tensile machine |
| Oxidative | b | Differential scanning calorimetry (DSC) |
| Chemical | c | Electron microscope and spectroscopy |
| Creep | d | Dynamic mechanical analysis (DMA) |

Table 1 shows an example set of $Q_{10}$ values of a medical plastic. These values are ordered into a hierarchy as defined by a set of rules identified by historic ageing test data which determine the overall $Q_{10}$ value of the medical plastic as c.

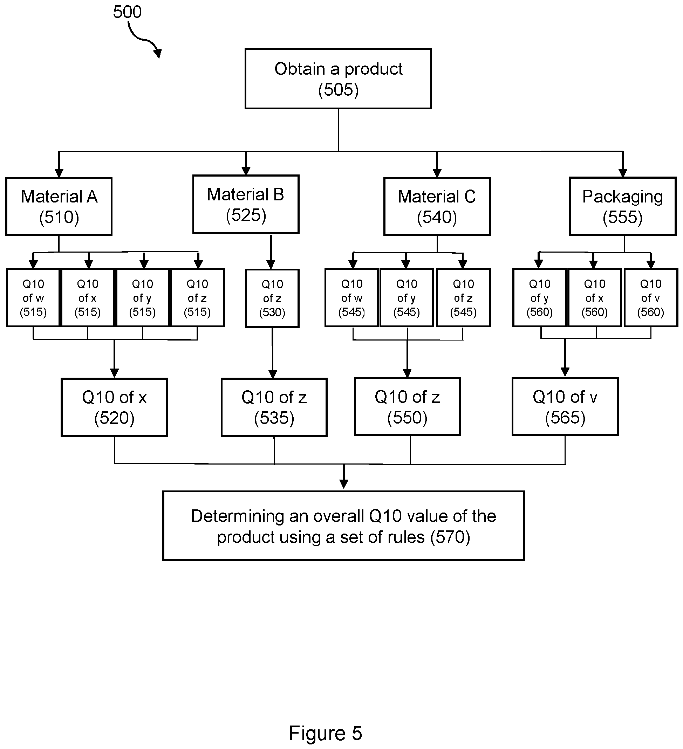

In use, using independent polymer analysis techniques, the first example computer-implemented method 200 of Figure 1 establishes reliable $Q_{10}$ values for each of the plurality of materials in the product and combines the different $Q_{10}$ values into a single, rational value to provide a conservative acceleration factor, or overall $Q_{10}$ factor, to give the shortest-possible accelerated ageing test with a comfortable margin of safety. The steps of determining a $Q_{10}$ value for a product from multiple sources of $Q_{10}$ values is illustrated more clearly in Figure 5.

Figure 3 shows a more detailed flow chart 300 of the first example computer-implemented method 200 that is depicted in Figure 2 with additional embodiments. The additional embodiments of Figure 2 allow the computer-implemented method 300 to obtain $Q_{10}$ values of each of the plurality of materials primarily from a database or secondarily by manual experiments and allow outputted values to be used in future runs of the computer-implemented method 300.

The example computer-implemented method 300 in Figure 3 is the same as the computer-implemented method 200 shown in Figure 2 but additionally comprises multiple steps for the step 210 of method 200. Instead of obtaining the $Q_{10}$ value of each of the plurality of materials of the product, based on the obtained parameters 210 method 300 comprises identifying materials in a database which comprise at least one of the obtained parameters, wherein the database comprises $Q_{10}$ values of materials 310, identifying if the parameters of the identified materials comprise a select threshold amount of the parameters of the plurality of materials of the product 315, and obtaining $Q_{10}$ values of materials in the database which comprise at least a select threshold amount of the parameters of the plurality of materials of the product 315 or obtaining $Q_{10}$ values manually for materials of the product that comprise less than a select threshold amount of the parameters of materials in the database 320 before determining an overall $Q_{10}$ value of the product, using a set of rules and the $Q_{10}$ values of the plurality of materials 325. After method 300 outputs an appropriate ageing temperature and the overall $Q_{10}$ value for an accelerated ageing test of the product to calculate the lifespan of the product 330, it comprises inputting the outputted appropriate ageing temperature and overall $Q_{10}$ value for an accelerated ageing test of the product into the database for use in further product analysis 335.

The database used in step 310 of computer-implemented method 300 optionally comprises the parameters, $Q_{10}$ values, and activation energy of previously analysed materials and ageing mechanisms in addition to other information, such as appropriate ageing conditions for each material or mechanism or the lifespan of each material. The database comprises information relating to, for example, engineering polymers, rubbers, multilayer films, and thermoplastic elastomers.

The selective threshold level used in steps 315 and 320 of computer-implemented method 300 is optionally influenced by the set of rules and defines the criteria that all materials in the database must meet in order to provide the $Q_{10}$ value for a material in the plurality of materials of the product. As a result, the selective threshold of steps 315 and 320 may optionally comprise a numeric requirement of likeness, for example, in polymer intrinsic properties, environmental conditions during the life cycle, and/or intended use of the product, depending on the material or product in question and the set of rules. In examples, a previous material of the database must comprise 98.5% of the composition of a material in the product to determine the $Q_{10}$ value of the material. In other examples, a previous material of the database comprises only 77% of the composition of a material in the product as the previous material undergoes 100% the same usage and faces 100% the same environmental factors in its life cycle. Therefore, the skilled person will understand that the selective threshold used by computer-implemented method 300, and further computer-implemented methods of the disclosure, may adapt to the requirements of the inputted parameters relating to the plurality of materials of the product 305 and the set of rules.

The database used in step 310 is configured to provide analysed materials for comparison against materials of the product, wherein if the parameters of a material are within the selected threshold level of likeness with a material of the product 315, the $Q_{10}$ value of the material in the database is used for step 325 in determining the overall $Q_{10}$ value of the product 335. If the parameters of all materials in the database are not within the selected threshold level of similarity with a material of the product, the $Q_{10}$ value of the material of the product is obtained manually through laboratory experiments 320. The manual obtaining of $Q_{10}$ values of a material in step 320 is undertaken by a separate method which is discussed in greater detail with reference to example method 400, shown in Figure 4A, or by other material testing techniques described with reference to Figures 14A, 14B, 15A, and 15B.

The feedback mechanism in step 335 is configured to insert any outputted $Q_{10}$ values and appropriate ageing test conditions 330 of the computer-implemented method 300 into the database of materials and $Q_{10}$ values used in step 310. In examples, the outputted $Q_{10}$ values and appropriate ageing test conditions also influence the set of rules, as well as expanding the database in step 335, for future decision making.

In use, the more detailed flow chart of the first example computer-implemented method 300 depicted in Figure 2 with additional embodiments calculates and identifies the accurate $Q_{10}$ value and the efficient appropriate ageing test conditions of a product, respectively, without excessive, and in some cases no, material testing.

Figure 4A shows a flow chart of an example method 400 used to manually determine the $Q_{10}$ factor of a material, for example one of the plurality of materials of a product. In this particular example 400, the $Q_{10}$ factor of the material is being assessed through tensile testing such as described in D20 Committee, Test Method for Tensile Properties of Plastics, ASTM D638-14, ASTM International (West Conshohocken, PA, 2014). This tensile testing example 400 comprises storing over 300 samples of the material in accelerated ageing chambers of different temperatures for different durations 405, removing the samples and conditioning them at room temperature (RT) for one to two hours 410, incrementally loading each sample and recording the strain of the material at each incremental load 415, obtaining material characteristics, for example stiffness and yield strength, for each aged sample 420, calculating the real time equivalent for the accelerated ageing duration of each sample using the Arrhenius equation 425, plotting the material characteristic against the logarithm of the real time 430, altering the $Q_{10}$ value in the real time calculation until the plot displays an R-squared value, or correlation of determination, of approximately 1 and has linear regression 435, and outputting the $Q_{10}$ value of the material 440. For clarity, the Arrhenius equation, shown above with reference to Figure 1, is rearranged to give the real time of the aged samples as

$$t_{RT} = t_{AA} Q_{10}^{\frac{T_{AA}-T_{RT}}{10}}.$$

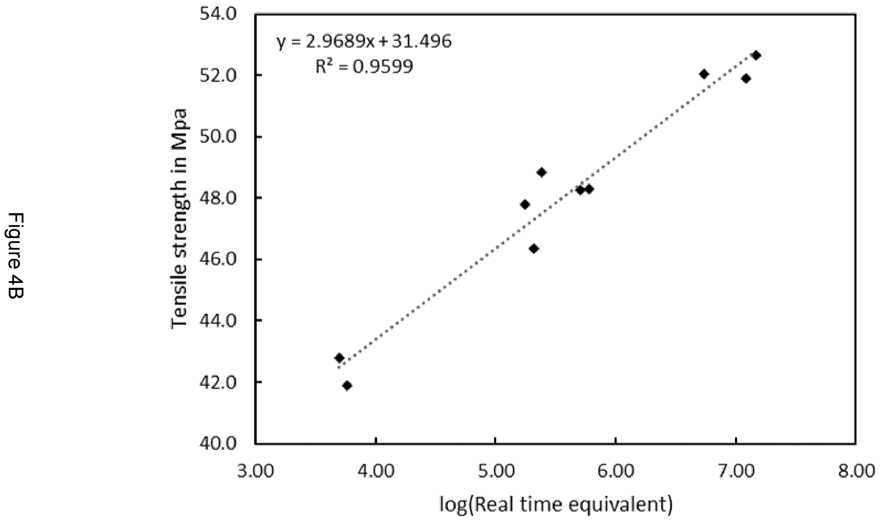

Figure 4B depicts steps 430 and 435 of method 400 wherein the averaged tensile strength values obtained from product samples are plotted against the logarithm of real ageing time and the $Q_{10}$ value is altered until the plot displays a correlation of determination close to 1. Figure 4B depicts the tensile strength of a product, Tritan MX731, and exhibits a largest correlation of determination of 0.9599. This correlation of determination corresponds to a value of 8.352 for the $Q_{10}$ tensile ageing factor of the product. In examples, the determination of the $Q_{10}$ value can optionally be achieved using the computer-implemented methods disclosed, by way of, for example, mathematical software, graphing calculators, or external online platforms.

The skilled person will understand that other tests, such as corrosion testing or creep testing, can be done to the same material to determine more ageing mechanisms, and their corresponding $Q_{10}$ values, which deteriorate the material. For example, in creep testing the material characteristic data, or TTS data, collected is the time in which each of the samples start to undergo irreversible deformation under a constant load. Examples of other material testing and $Q_{10}$ obtaining techniques are described below with reference to Figures 14A, 14B, 15A, and 15B.

In examples, in all the computer-implemented methods of the present disclosure, any $Q_{10}$ values of 2 obtained manually or through a database are optionally disregarded. $Q_{10}$ of 2 values represent oxidation and ageing mechanisms of semicrystalline polymer chains which are rarely the fastest or most detrimental ageing mechanism occurring in a product.

Traditional accelerated ageing studies use the default conservative $Q_{10}$ ageing value of 2 as based on the general guidance from ASTMD1980F. This approach requires excessively long accelerated ageing tests to replicate long-term performance of a product, as predicted by the Arrhenius equation, and leads to unnecessarily over-aged devices which may fail ageing tests or provide an underestimation of the product shelf-life. Most materials and products, when in use, face faster acting ageing mechanisms that pose greater threat to the integrity of the material or product; disregarding $Q_{10}$ of 2 values therefore reduces the spread of $Q_{10}$ values and improves the accuracy of determining the overall $Q_{10}$ value of a product.

The step 405, in which over 300 samples of the material are stored in accelerated ageing chambers of different temperatures for different durations, can optionally be done in multiple successive temperature chambers with multiple test durations. In this case the above real time equation would become

$$t_{RT} = t_{AA1}Q_{10}^{\frac{T_{AA1}-T_{RT}}{10}} + t_{AA2}Q_{10}^{\frac{T_{AA2}-T_{RT}}{10}} + \cdots + t_{AAn}Q_{10}^{\frac{T_{AAn}-T_{RT}}{10}}.$$

Furthermore, the recording of data done in step 415 may optionally comprise using the computer-implemented method 300 of the disclosure. Likewise, the steps for plotting the material characteristic against the logarithm of the real time 430 and altering the $Q_{10}$ value in the real time calculation until the plot displays an R-squared value of approximately 1 and has linear regression 435 could be done, for example, by utilising the computer-implemented method 300 or another programme which inputs the final results to the computer-implemented method 300 when the $Q_{10}$ value of each of the plurality of materials of the product are being obtained in steps 210 and 320 of example computer-implemented methods 200 and 300.

In use, procedure 400 taken to manually determine the $Q_{10}$ factor of a material outputs accurate $Q_{10}$ values of a material which may be used to determine an appropriate accelerated ageing test duration and determine the lifespan of a product reliably and cost and time efficiently.

Table 2 below shows an example table which may be used to display the ageing mechanisms, and the corresponding $Q_{10}$ values, that occur in each material of a product and are obtained by methods such as 400. The table comprises multiple $Q_{10}$ values of materials, either obtained through the database of $Q_{10}$ values or by manual testing, to depict the different rates of ageing mechanisms acting on the materials. A table like this, displaying multiple $Q_{10}$ values of multiple materials, is optionally used by the example computer-implemented methods 200, 300 of Figures 2 and 3, with assistance from the set of rules, for obtaining the $Q_{10}$ value of each of the plurality of materials of a product 210, 315, 320 and for determining an overall $Q_{10}$ value of the product 215, 325.

| Material | $Q_{10}$ physical ageing | $Q_{10}$ oxidation ageing | $Q_{10}$ creep |
|---|---|---|---|
| 1 | A | B | C |
| 2 | X | Y | Z |

Table 2 shows multiple ageing mechanism and their $Q_{10}$ values for two materials of a product.

Figure 5 shows a flow chart 500 of how the first example computer-implemented method 200, 300 determines the overall $Q_{10}$ value of a product from multiple $Q_{10}$ values of the plurality of materials of the product, using a set of rules. This procedure comprises obtaining a product 505 and identifying the component materials of the product 510, 525, 540, 555, identifying the ageing mechanisms occurring in each product 515, 530, 545, 560, obtaining the dominant $Q_{10}$ value of each material 520, 535, 550, 565 and determining an overall $Q_{10}$ value of the product using a set of rules 570. In examples, the $Q_{10}$ value of each material can be determined either manually by, for example, a method 400 as shown in Figure 4A or via a database, for example, as shown in method 300 of Figure 3. As shown in Figure 5, the materials of the product may comprise a broad range of components including, but not limited to, abundant component materials, joining mechanisms, packaging, and wiring. In examples, the set of rules discounts any material that is not a polymer as these materials do not deteriorate relatively fast. The set of rules are configured to determine the fastest ageing mechanism or most dominant ageing mechanism of the product as discussed with reference to Figure 1. Optionally, prior to steps 510, 525, 540, 555 wherein the component materials of the product are identified, the method of determining the overall $Q_{10}$ value of a product comprises checking the product for environmental stress cracking (ESC), wherein if ESC is present the product is rejected and requires ESC prevent methods. If ESC is not present in the product, the method undergoes identifying the component materials of the product at steps 510, 525, 540, 555. The presence of ESC is detrimental to any material and will indubitably cause imminent product failure.

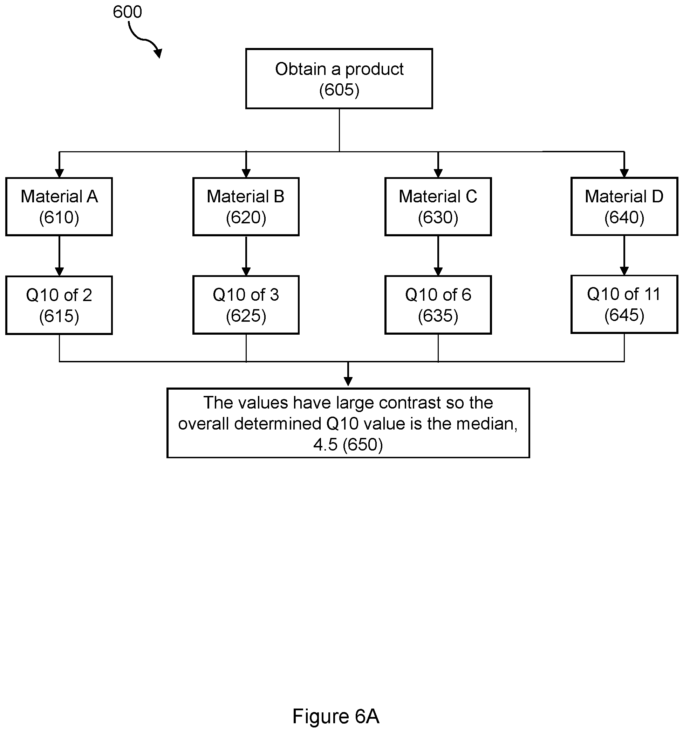

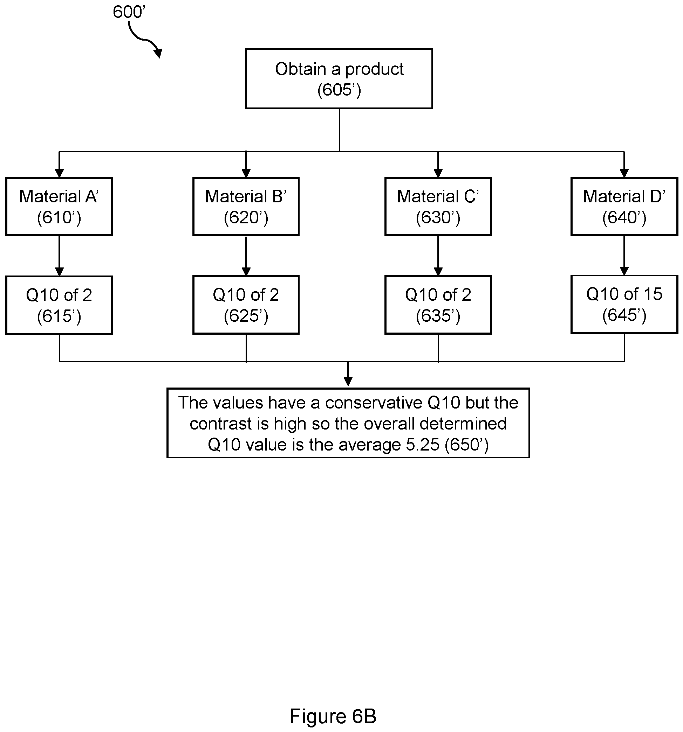

Figure 6A and 6B show worked examples of flow chart 500 in Figure 5 wherein the overall $Q_{10}$ value of products is determined. Each example comprises a product 605, 605' comprising four materials 610, 620, 630, 640, 610', 620', 630', 640' that each have a $Q_{10}$ value 615, 625, 635, 645, 615', 625', 635', 645', respectively. The examples then comprise determining the overall $Q_{10}$ value of the product 650, 650' using the set of rules described with reference to Figure 1.

The first worked example 600, shown in Figure 6A, comprises four materials A 610, B 620, C 630, and D 640, which each comprise a $Q_{10}$ value of 2, 3, 6, and 11. In this first example the set of rules identify the spread of values to be large and the overall $Q_{10}$ value of the product to be the median, 4.5. The second worked example 600', shown in Figure 6B, comprises four materials A' 610', B' 620', C' 630', and D' 640', which each comprise a $Q_{10}$ value of 2, 2, 2, and 15. In this first example the set of rules identify the spread of values to be conservative but the contrast of such spread to be high. As a result, the overall $Q_{10}$ value of the product in Figure 6B is taken as the average, 5.25. In these examples the statistic reasoning of the set of rules are utilised without the need for historic ageing data. In examples where the materials and the product usage is known, historic ageing data may be appropriate for the of rules decision.

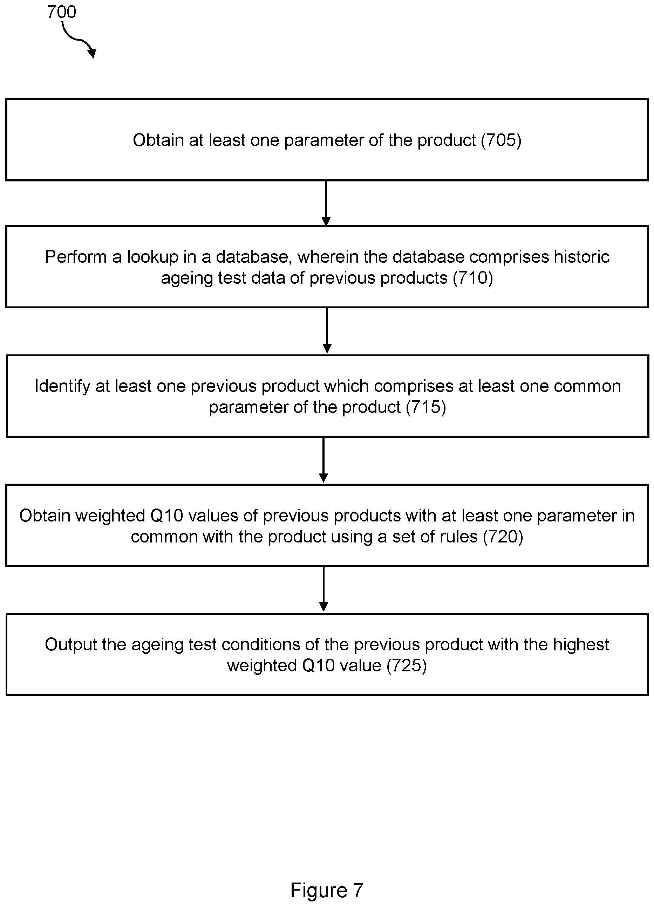

Figure 7 shows a flow chart of a second example computer-implemented method 700 of the present disclosure which could be used in the example method 100 of Figure 1. In this example, the computer-implemented method 700 comprises obtaining at least one parameter of the product 705, as described with reference to Figure 1, performing a lookup in a database, wherein the database comprises historic ageing test data of previous products 710, identifying at least one previous product in the database which comprises at least one common parameter of the product 715, obtaining weighted $Q_{10}$ values of previous products with at least one parameter in common with the product using a set of rules 720, and outputting the ageing test conditions of the previous product with the highest weighted $Q_{10}$ value 725. The features which differentiate this method 700 to that of Figures 2 and 3 are the quantification of the product as a whole, rather than in its component materials, the database comprising historic ageing test data of previous products, the identification of previous products alike to the product being analysed through at least one common parameter as opposed to a threshold of similarity, and the weighting mechanism of the $Q_{10}$ values obtained using the set of rules.

The parameters obtained in step 705 may comprise any of those discussed in the method of Figure 1. The lookup performed in the database in step 710 may comprise a lookup of the at least one parameter of the product by way of part names and classification, wherein the classifications optionally include, but are not limited to, the intended use of the product, the factors relating to the environment, and the polymer intrinsic properties of the plurality of materials. The classifications and part names comprise groups of products, wherein the products have identified accurate $Q_{10}$ values and appropriate ageing test conditions. Furthermore, the step of identifying at least one previous product which comprises at least one common parameter of the product 715 comprises filtering all the previous products in the database into a small select group, wherein the products are filtered into the small select group if they are highlighted by the lookup of step 710 to comprise at least one parameter in common with the product being analysed by the method. In examples, smaller selected groups are created from the previous products in the database, wherein the products are filtered into increasingly smaller select groups if they have, for example, at least two, three, four, or five parameters in common with the product being analysed by the method, respectively. In embodiments, step 720 of obtaining weighted $Q_{10}$ values of previous products with at least one parameter in common with the product using a set of rules comprises obtaining a weighted $Q_{10}$ value of each previous product in the small select group using a set of rules, wherein each previous product in the smaller select group has multiple weighted $Q_{10}$ values, and wherein obtaining the most appropriate weighted $Q_{10}$ value for each previous product with at least one parameter in common with the product using the set of rules 720 is based on the influential factors of the set of rules. For example, the set of rules are optionally influenced by the same factors or historic data as discussed in Figure 1. In other embodiments, step 720 of obtaining weighted $Q_{10}$ values of previous products with at least one parameter in common with the product using a set of rules 720 comprises obtaining, from the database, a $Q_{10}$ value of each previous product from the smaller select group and assigning each $Q_{10}$ value an appropriate weighting using the set of rules. The final step 725, of outputting the ageing test conditions of the previous product with the highest weighted Q10 value 725, comprises either outputting the highest weighted $Q_{10}$ value that was obtained using the set of rules or outputting the obtained $Q_{10}$ value which was weighted the highest by the set of rules.

In computer-implemented method 700 of Figure 7 the step of obtaining at least one parameter of the product 705 is configured to quickly quantify the product, wherein the accuracy of quantifying the product is improved by obtaining increasingly more parameters of the product. The step of performing a lookup in a database, wherein the database comprises historic ageing test data of previous products 710 is configured to negate the need for manual testing and provide historic ageing test data for previous which comprises appropriate ageing test conditions and $Q_{10}$ values that are appropriate for reuse in an ageing test of the product. Moreover, the step of identifying at least one previous product which comprises at least one common parameter of the product 715 is configured to narrow down the appropriate $Q_{10}$ values and historic ageing test data that are appropriate for the ageing test of the product. Step 720 of obtaining weighted $Q_{10}$ values of previous products with at least one parameter in common with the product using a set of rules is configured to select the most appropriate $Q_{10}$ value and historic ageing test data for the accelerated ageing test of the product. Lastly, step 725 of outputting the ageing test conditions of the previous product with the highest weighted $Q_{10}$ value is configured to provide the information needed for undertaking the accelerated ageing test of the product.

In use, the second example computer-implemented method 700 of Figure 7 allows the lifespan of a product to be calculated by outputting accurate accelerated ageing test conditions. The method 700 outputs the accurate conditions needed without the need for manual material testing and without the need to analyse each material of the product individually.

Traditionally, as mentioned previously, manufacturers take the $Q_{10}$ value of any material to be 2. This assumption reduces the need for ageing mechanism testing but causes excess consumption of effort and energy, significantly increases the carbon footprint in product testing, and delays product release to the market by several years. The disclosed computer-implemented method 700, for example, can reduce the accelerate ageing test duration by up to six times that of conventional methods. Previous accelerated ageing test durations of three different materials using this accurate computer-implemented method and conventional methods are depicted below in table 3.

| Product | Accelerated ageing time based on $Q_{10}$ of 2 | Accelerate ageing test based on $Q_{10}$ evaluated by computer-implemented method |
|---------|------------------------------------------------|-----------------------------------------------------------------------------------|
| A | 337 days | 71 days |
| B | 238 days | 52 days |
| C | 238 days | 38 days |

Table 3 shows the difference in accelerated ageing test durations for $Q_{10}$ values determined using the conventional method, where the $Q_{10}$ value is taken to be 2, and the disclosed accurate computer-implemented method 700.

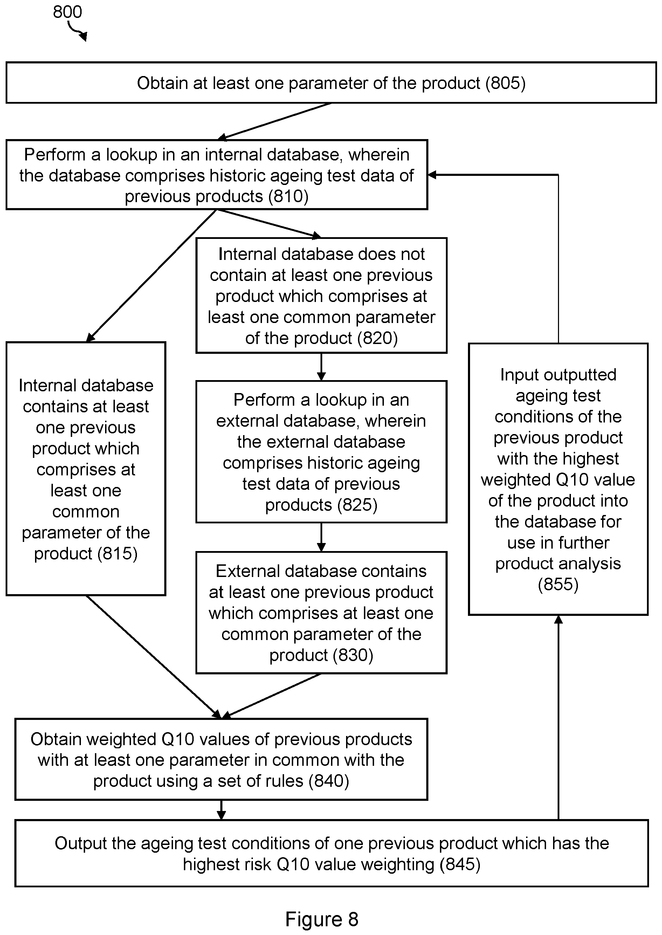

Figure 8 shows a more detailed flow chart of the second example computer-implemented method 800 that is depicted in Figure 7 with additional embodiments. The second example method 800 in Figure 8 is the same as that shown in Figure 7 but comprises a more complex step 715, wherein at least one previous product which comprises at least one common parameter of the product is identified. Instead example computer-implemented method 800 comprises performing a lookup in an internal database, wherein the database comprises historic ageing test data of previous products 810. If the internal database contains at least one previous product which comprises at least one common parameter of the product 815 then computer-implemented method 800 comprises obtaining weighted $Q_{10}$ values of previous products with at least one parameter in common with the product using a set of rules 840. If the internal database does not contain at least one previous product which comprises at least one common parameter of the product 820 then the computer-implemented method 800 comprises performing a lookup in an external database, wherein the external database comprises historic ageing test data of previous products 825 and obtaining weighted $Q_{10}$ values of previous products with at least one parameter in common with the product using a set of rules 840 if the external database contains at least one previous product which comprises at least one common parameter of the product. As with computer-implemented method 700, computer-implemented method 800 comprises outputting the ageing test conditions of one previous product which has the highest risk $Q_{10}$ value weighting 845 regardless of which database provided the $Q_{10}$ values.

The second example method 800 in Figure 8 also comprises inputting outputted ageing test conditions of the previous product with the highest weighted $Q_{10}$ value of the product into the database for use in further product analysis 855. In examples, wherein no previous product comprises at least one common parameter of the product is identified in the internal or external database, the $Q_{10}$ value of the product may be obtained manually through experiment and be outputted with appropriate ageing test conditions. This manual experimentation would replace steps 840 and 845.

Step 825 of performing a lookup in an external database, wherein the external database comprises historic ageing test data of previous products is configured to provide more historic ageing test data in case the internal database comprises no previous product which comprises at least one common parameter of the product. Furthermore, the step of feeding back outputted ageing test conditions of the previous product with the highest weighted $Q_{10}$ value of the product into the memory of the database 855 is configured to expand the database so that the step of identifying previous products in the future is quicker and more reliable and the outputted ageing test conditions are more accurate.

The second example computer-implemented method 800 of Figure 8 has the same use of computer-implemented method 700 in Figure 7 with additional steps to overcome the rare occurrence that the database does not contain information required for outputting the ageing test conditions of one previous product which has the highest risk $Q_{10}$ value weighting 845.

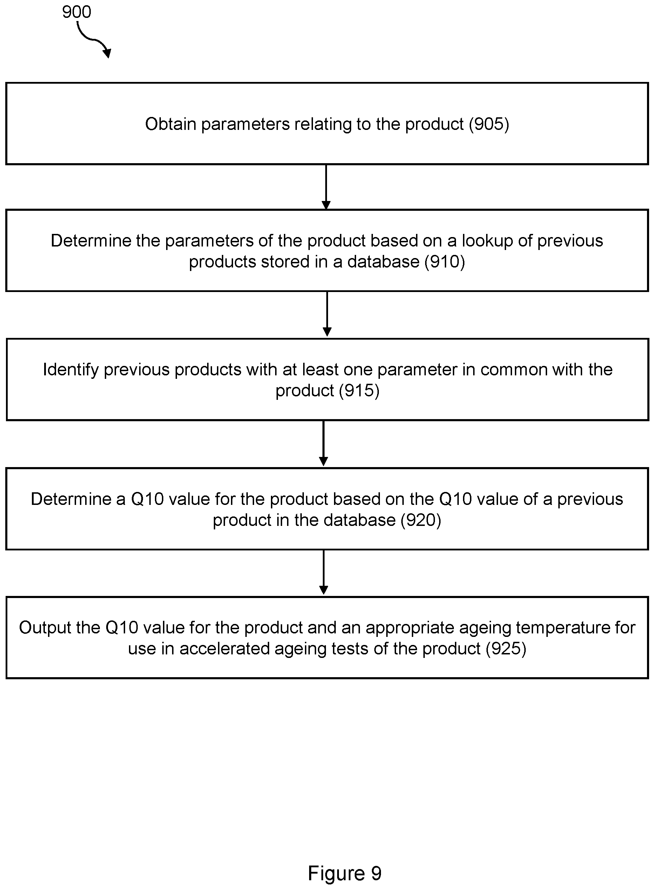

Figure 9 shows a flow chart of the third example computer-implemented method 900 of the present disclosure which could be used in the example method 100 of Figure 1. The third example computer-implemented method 900 comprises obtaining parameters relating to the product 905, determining the parameters of the product based on a lookup of previous products stored in a database 910, identifying previous products with at least one parameter in common with the product 915, determining a $Q_{10}$ value for the product based on the $Q_{10}$ value of a previous product in the database 920, and outputting the $Q_{10}$ value for the product and an appropriate ageing temperature for use in accelerated ageing tests of the product 925. The database of step 910 is abundant and optionally comprises significant data about a wide range of products and materials wherein data acquisition occurs, for example, through manual experimentation or procurement from accredited sources.

This third example computer-implemented method 900 of Figure 9 is similar to the second example computer-implemented method 700 of Figure 7 without comprising weighted $Q_{10}$ values and a set of rules. The computer-implemented method 900 of Figure 9 does not comprise weighted $Q_{10}$ values as the computer-implemented method 900 comprises obtaining multiple parameters of the product 905. By obtaining multiple parameters of the product 905, the lookup in the database 910 may identify previous products with more than one parameter in common with the product; these identified previous products 915 therefore are ideally almost replicas of the product. The determined and outputted $Q_{10}$ value for the product 920, 925 is the value of the previous product identified from the database. The method 900 of Figure 9 is configured to output a $Q_{10}$ value and appropriate ageing test conditions for a product 925 without using a set of rules.

In use, computer-implemented method 900 of Figure 9 quickly outputs an accurate $Q_{10}$ value and appropriate ageing temperature for a product provided that adequate information about the product is known.

Figure 10 shows a more detailed flow chart of the third example computer-implemented method 1000 that is depicted in Figure 9 with additional embodiments. The computer-implemented method 1000 differentiates to the computer-implemented method 900 shown in Figure 9 as it has a more complex step 915, wherein previous products with at least one parameter in common with the product are identified, and a more complex step 920, wherein the $Q_{10}$ value for the product based on the $Q_{10}$ value of a previous product in the database is determined. In computer-implemented method 1000 there are three methods to get from determining the parameters of the product based on a lookup of previous products stored in a database 910 to outputting the $Q_{10}$ value for the product and an appropriate ageing temperature for use in accelerated ageing tests of the product 925. Furthermore, there is also an additional step in computer-implemented method 1000 that is not present in computer-implemented method 900. This additional step comprises inputting the $Q_{10}$ value for the product and an appropriate ageing temperature for use in accelerated ageing tests of the product back into the internal database for use in further product analysis 1055.

The first, most preferential, method of the three previously mentioned methods to get from step 910 to 925 comprises identifying at least one previous product in an internal database comprising a select threshold amount of the parameters of the product 1015 then determining a $Q_{10}$ value for the product based on the $Q_{10}$ value of a previous product in the database 1020. The second most preferential method of the three previously mentioned methods to get from step 910 to 925 comprises identifying no previous products in the internal database comprising a select threshold amount of the parameters of the product 1025, determining the parameters of the product based on a lookup of previous products stored in an external database 1030, identifying at least one previous product in the external database comprising a select threshold amount of the parameters of the product 1035, and determining a $Q_{10}$ value for the product based on the $Q_{10}$ value of a previous product in the database 1020. The least preferential method of the three previously mentioned methods to get from step 910 to 925 comprises identifying no previous products in the internal database comprising a select threshold amount of the parameters of the product 1025, determining the parameters of the product based on a lookup of previous products stored in an external database 1030, identifying no previous products in the external database comprising a select threshold amount of the parameters of the product 1040, and obtaining a $Q_{10}$ value for the product manually 1045, wherein manually obtaining the $Q_{10}$ value of the product comprises performing a method similar to the example method 400 shown in Figure 4A.

The parameters, internal database, look up mechanism, and selective threshold seen in computer-implemented method 1000 are synonymous with those of computer-implemented methods 200, 300, 600, 700, and 800. As previously discussed, the parameters obtained by the computer-implemented method 1000 comprise characteristics relating to the product, the internal database optionally comprises parameters, $Q_{10}$ values, and appropriate ageing test conditions of previously analysed products, the look up mechanism optionally uses part names and classifications, and the selective threshold optionally comprises a varying numeric value depending on the obtained parameters of the product.

The external database in steps 1040, 1035, and 1040 of computer-implemented method 1000 optionally comprises parameters, $Q_{10}$ values, lifespan and appropriate ageing test conditions of previously analysed products. The external database may comprise some of the previously analysed products in the internal database however, due to the hierarchical nature of the three methods between determining the parameters of the product 1010 and outputting the $Q_{10}$ value and appropriate ageing test conditions 1050, the lookup will be performed in the internal database and the materials present in the internal database will be utilised before performing the same lookup in the external database.

The three different methods in computer-implemented method 1000 used to output the $Q_{10}$ value for the product and an appropriate ageing temperature 1050 are configured to provide the necessary data needed to calculate the lifespan of a product such as in method 100 in Figure 1. The first most preferential method is configured to output the $Q_{10}$ value and appropriate ageing temperature of a previous product stored in the internal database with the selective threshold amount of the obtained parameters of the product. The second preferential method is followed when the selective threshold in the first method is not met. This second method is configured to output the $Q_{10}$ value and appropriate ageing temperature of a previous product stored in the external database with the selective threshold amount of the obtained parameters of the product. The third, least preferential method, is followed when neither databases comprise a previous product stored in the with the selective threshold amount of the obtained parameters of the product. The third method is configured to manually obtain, by experimental tests, and output the $Q_{10}$ value and appropriate ageing temperature for use in accelerated ageing tests of the product.

In use, computer-implemented method 1000 outputs the data needed to undertake accelerated ageing tests of products. The computer-implemented method 1000 may provide fast data results using previous product data. The method 1000 also allows for the case where previous product data is not available and initiates the use of experimental methods to provide such data.

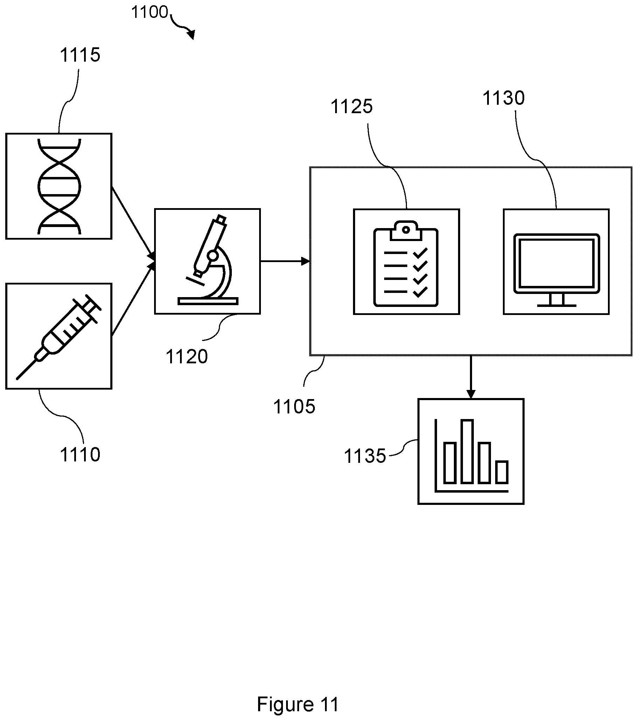

Figure 11 shows an example system 1100 configured to perform the first example computer-implemented method 200 of the present invention and provide context for the use of computer-implemented method 200 of Figure 2. The example system 1100 comprises multiple devices 1110, information about the parameters of the devices 1115, a processor 1105, experimental tests 1120, and outputted data about the devices 1135. The multiple devices 1110 optionally comprise components of a larger product or, for example, one time use devices used in the same procedure, such as a medical procedure or aerospace testing. The parameters of the devices 1115 comprise those discussed with reference to Figure 1 such as the polymer intrinsic structures. The processor 1105 comprises a set of rules 1125 and a large language model 1130, wherein the set of rules 1125 are influenced by data or statistical reasoning as discussed in Figure 1 and wherein the large language model 1130 comprises summarising the experimental test results 1120, predicting multiple $Q_{10}$ values of the devices from the experimental test results 1120 or the environmental factor and intended use parameters and outputting, using the set of rules 1125, the appropriate ageing temperature and overall $Q_{10}$ value for an accelerated ageing test of the product or devices to calculate the lifespan of the product. The outputted data 1135 about the devices optionally comprises the overall $Q_{10}$ value, appropriate accelerated ageing temperature, and appropriate accelerated ageing duration.

The experimental test 1120 of system 1100 is configured to analyse the devices 1110 and the parameters of the device 1115 to provide the processor with material characteristics of multiple aged samples of each of the plurality of materials of the device. The large language model 1130 of the processor 1105 obtains and uses the material characteristics of the multiple aged samples to generate $Q_{10}$ values of each of the plurality of materials of the device. The set of rules 1125 of the processor 1105 are then configured to rank the $Q_{10}$ values into a hierarchy and determine the overall $Q_{10}$ value which poses the most risk to the integrity of the devices or product 1110. The outputted data 1135 is configured to provide the means necessary to undergo accelerated ageing tests of the devices or product 1110 to calculate the lifespan.

In use, system 1100 turns devices and information about the devices into numerical values that can be used to calculate the lifespan of the devices.

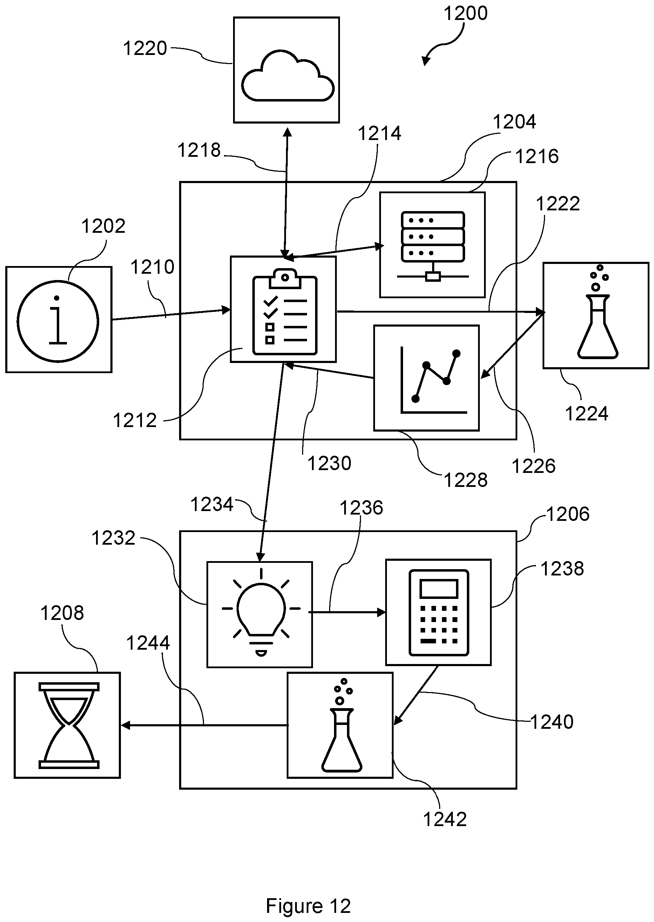

Figure 12 shows an example system 1200 which utilises the computer-implemented method of the present invention to calculate the lifespan of a product. System 1200 provides the context in which computer-implemented methods 300, 700, 800, 900, and 1000 optionally sit and interact with other components to provide the lifespan of a product.

System 1200 comprises multiple features and multiple processes. The features of system 1200 comprise parameters of the product 1202, a processor 1204, an external database 1220, experimental $Q_{10}$ testing 1224, accelerated ageing testing 1206 and the lifespan of a product 1208. The processor of system 1200 comprises a set of rules 1212, previously described with reference to Figure 1, an internal database 1216, previously described with reference to Figure 3, and a data recording module 1228. The accelerated ageing testing 1206 comprises a $Q_{10}$ value for the product and an appropriate ageing temperature for use in accelerated ageing tests of the product 1232, a calculation module 1238, and an experimental accelerated age test 1242.

The processes of the system 1200 comprise inputting parameters relating to the product into the processor to be read by the set of rules 1210, identifying at least one previous product in the internal database comprising a select threshold amount of the parameters of the product 1214, identifying at least one previous product in the external database comprising a select threshold amount of the parameters of the product 1218, identifying no previous products in the external or internal databases comprising a select threshold amount of the parameters of the product and instructing an experimental test 1222, outputting manually obtained material characteristics for aged samples of the product 1226, inputting manually obtained $Q_{10}$ values of the product into the set of rules 1230, outputting an overall $Q_{10}$ value and appropriate ageing temperature for an accelerated ageing test of the product 1234, transfer of data 1236, outputting the appropriate accelerated ageing duration of the product, and outputting the determined lifespan of the product 1244.

The processor 1204 is configured to receive parameters relating to the product and identify a $Q_{10}$ value for the product which is outputted for an accelerated ageing test to calculate the lifespan of the product. The processor works with an external database 1220 and laboratory experiments to obtain the $Q_{10}$ value either automatically, from previous product analysis, or manually, from testing if it does not already comprise the $Q_{10}$ value in the internal database. The set of rules 1212 are configured to order the risk of the multitude of $Q_{10}$ values that may be obtained for the product. The set of rules 1212 are then configured to determine an overall $Q_{10}$ value of the product and output this $Q_{10}$ value, with the appropriate ageing temperature, to the appropriate ageing test 1206.

The appropriate ageing test 1206 is configured to receive an overall $Q_{10}$ value of the product and appropriate ageing temperature and, using this information, calculate an appropriate ageing test duration 1238. The appropriate ageing test 1206 is then configured to undergo experimental appropriate ageing tests 1242 to determine and output the lifespan of the product 1208.

In use, system 1200 receives information about a product and outputs an accurate lifespan prediction of the product.

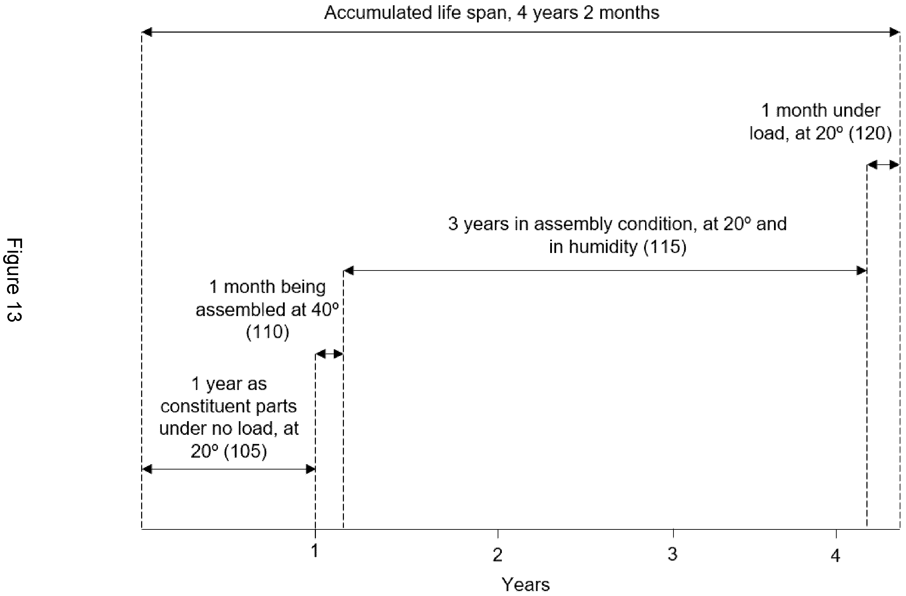

Figure 13 shows a graph depicting the life cycle of an example product which may be analysed by the computer-implemented methods of Figures 2, 3, 7, 8, 9, and 10. The life cycle of a product comprises constituent parts which reflect when a product, for example, is manufactured, sterilised, shipped, stored, and used. In the example shown in Figure 10 the product has an accumulated lifespan of 4 years and 2 months which is made up of 1 year as constituent parts, 1 month being assembled, 3 years being stored, and 1 month in use. When determining either the total lifespan of a product throughout its whole life cycle, the lifespan of a product for a part of its life cycle, or the shelf-life of the product, the parameters relating to the product are required, as discussed with reference to Figure 1. In this example, the 1 month of assembly is undertaken at 40 degrees Celsius. If an identical product has the same life cycle but is assembled instead at 20 degrees Celsius, it would be expected that the chemical and mechanical processes occurring in the products acted at a different rate and produce different $Q_{10}$ values. The shelf-life of the two near identical products and life cycles will be disparate.

The present disclosure will now describe more example ways of determining the Q10 of a product or material.

Method 400, shown in Figure 4A, shows how the physical ageing $Q_{10}$ value for a material is determined by a tensile test. This example method 400 depicts a typical mechanical material test which may take weeks or months to collect data. Another example technique, called DMA, used to determine the $Q_{10}$ value of a material or product is depicted with reference to Figures 14A and 14B. DMA is a technique also used to study the mechanical properties of materials as a function of temperature, time, frequency, stress, or other variables while subjecting them to a dynamic mechanical load. In this technique, collected data, comprising a single curve of a material characteristic at every temperature increment, will be shifted across a logarithmic time axis to create a final creep master curve of the material. In examples, DMA software, optionally incorporated into the computer-implemented methods of the present disclosure, completes this shift automatically and yields the activation energy (Ea) of relaxation which can be used in the following iteration of the Arrhenius equation:

$$K_T = Ae^{\frac{-Ea}{RT}}.$$

In this equation, $K_T$ is the rate constant, A is the pre-exponential factor, Ea is the activation energy, R is the universal gas constant, T is the absolute temperature.

DMA testing comprises ordering the materials of the product, producing matched dimensions of the DMA samples using the standard test method, ASTM D4065, developed by ASTM International for determining the tensile properties of fiber-reinforced plastic materials, undertaking TTS creep testing on the samples, analysing the data from the test, and determining the $Q_{10}$ value.

Tensile testing, as shown in method 400 of Figure 4, is a static or quasi-static test method for the characterization of the mechanical behaviour of plastics. The test provides details on the young's modulus and tensile strength of a material and is performed according to the standard ASTM D638. For the execution of the tensile test on plastics, different test specimens, so called "dogbones" are used and the material parameters such as tensile stress, tensile strength and elongation at break are be determined. Alternatively, materials, such as polymers, show a time dependent creep deformation under constant load. This occurs because the polymer under load undergoes molecular rearrangement to minimize the localized stresses. This deformation is dependent on the load case, applied stress, temperature, chemical environment, and time and this creep behaviour can be tested and quantified by, for example, TTS creep experiments.

Creep experiments are another manual test configured to determine the strain of the material under constant stress or the release of stress under constant strain. To accurately evaluate material performance for a specific application, one needs to test the material under the actual temperature and time conditions, the material will undergo during the end-use. Fortunately, that type of tedious testing, such as in method 400, is not necessary and instead accelerated temperature measurements and theoretical TTS treatment of the data using limited lab tests are sufficient to project long-term properties under a variety of conditions. DMA measures the modulus (stiffness) and damping (energy dissipation) properties of a material as the material is deformed under constant stress is one of the best thermal analysis techniques for using the TTS predictive approach. The underlying basis for TTS is the demonstrated equivalency between time (or frequency) and temperature. The superposition principle is based upon the premise that the processes involved in molecular relaxation or rearrangements occur at greater rates at higher temperatures. The time over which these processes occur can be reduced by conducting the measurement at elevated temperatures and transposing the data to lower temperature. It has been demonstrated that viscoelastic data collected at one temperature can be superimposed upon data obtained at a different temperature simply by shifting one of the curves along the time axis.

The viscoelastic data can be collected by performing static measurements under isothermal conditions (e.g., creep or stress relaxation) and the individual data at all ageing temperatures are used to construct a master curve. For the individual data sets, the lowest ageing temperature is used as the reference temperature and the experimental times at this temperature are multiplied by a constant shift factor using the Williams Landel Ferry equation,

$$\ln(a_T) = \frac{a(T - T_0)}{b + (T - T_0)},$$

Where $a_T$ is the shift factor of an isotherm determined at temperature T in relation to the isotherm at the reference

temperature $T_0$ and a and b are coefficients dependent on the material. As a result, the TTS method gives the most reliable test $Q_{10}$ value results since there are fewer stages of data manipulation compared to conventional methods such as that shown in Figure 4A.

The activation energy for the relaxation process run by DMA creep or stress relaxation experiments at constant stresses or strains is directly proportional to the physical aging $Q_{10}$ values of the materials. The higher the activation energy for relaxation, the higher the $Q_{10}$ value will be. In certain cases, if there is a reference reliable $Q_{10}$ value and master curve with the Ea of the relaxation process for a specific material, various other material's $Q_{10}$ value's can be quickly determined by running DMA creep or stress relaxation experiments and generating the master-curve with the TTS technique.

An example of this TTS principle is shown in Figure 14A and 14B. Figure 14A shows 32 MPa creep curves of two samples of a product that were aged at different temperatures, 30 and 60 degrees Celsius, for a maximum of 1000hrs (6 weeks) and Figure 14B shows the high temperature master curve shifted on the time axis to give the long term behaviour (over 2 years) of the product as it faces a constant applied stress of 32 MPa.

Figure 15A shows an example of a TTS creep master curve obtained for a product which is a film named S80. The point of interest within the graph used to determine the $Q_{10}$ value of the product is where there is a significant increase in the gradient, in other words where the strain in the sample reaches levels where it can be considered as 'permanent deformation'. This is the point at which creep is determined to have begun in earnest. For S80 in Figure 15A, this point is taken to be between $1 \times 10^2$ seconds and $1 \times 10^3$ seconds. From this a $Q_{10}$ ageing factor of 2.5 for S80 is calculated.

Figure 15B shows another example of a TTS creep master curve obtained for a product called Elastosil LR 3003/50. Here, the creep begins in earnest straight away, and the units of time are extremely low. This indicates that ageing will not be a concern for the silica components when compared to the hard plastics, and they will not need to be considered in ageing experiments.

The disclosure has discussed two methods, mechanical testing 400 and DMA, for determining the $Q_{10}$ value of a product wherein DMA testing is faster than mechanical testing and involves less mathematical manipulation of obtained data. Another example method, using DSC, utilises the ratio of semicrystalline chain structure to amorphous chain structure present in a product to obtain an overall $Q_{10}$ value of products, utilising the disclosed computer-implemented methods 200, 300, 700, 800, 900, and 1000, faster than DMA or mechanical testing. The fastest ageing mechanism, a physical ageing mechanism, happens in the amorphous chains of each polymer. If a scientist calculates the amorphous to semicrystalline ratio of the polymers with a DSC, they can estimate other reaction rates and $Q_{10}$ ageing values of various mechanisms. By studying one polymer and obtaining its $Q_{10}$ ageing value as a reference, the established amorphous-semicrystalline ratio can be applied to calculate the other $Q_{10}$ ageing values. Hence the $Q_{10}$ values for many mechanisms and many polymers may be determined without having to spend months and years of testing.

All the discussed techniques, for example mechanical testing, DMA, and DSC, can optionally be used in the computer-implemented methods 200, 300, 700, 800, 900, and 1000, to accurately determine the lifespan of a product.

The computer-implemented methods discussed with reference to Figures 1 to 15B are all configured to determine the lifespan of a product. The computer-implemented methods can be used to determine the overall $Q_{10}$ value and lifespan for the whole life cycle or parts of the life cycle of a product given that the obtained parameters relating to the materials of the product correspond to the whole life cycle or the part of the life cycle of interest.

It will be appreciated from the discussion above that the embodiments shown in the figures are merely exemplary, and include features which may be generalised, removed, or replaced as described herein and as set out in the claims. In the context of the present disclosure other examples and variations of the apparatus and methods described herein will be apparent to a person of skill in the art. For example, the present disclosure describes embodiments for calculating the lifespan and identifying the ageing test conditions for accelerated ageing tests of a product however it will be appreciated that other products, devices, components or materials may also be suitable for calculation and identification such as, but not limited to, polymers, pharmaceutical compounds, metals, skin samples, and combinations thereof.

<u>CLAUSES:</u>

1. A computer-implemented method for identifying the ageing test conditions for accelerated ageing tests of a product, wherein the method comprises:

obtaining at least one parameter of the product;
performing a lookup in a database, wherein the database comprises historic ageing test data of previous products;
identifying at least one previous product in the database which comprises at least one common parameter of the product;
obtaining weighted $Q_{10}$ values of previous products with at least one parameter in common with the product using a set of rules; and
outputting the ageing test conditions of the previous product with the highest weighted $Q_{10}$ value.

2. The computer-implemented method of clause 1 wherein the ageing test conditions comprise an appropriate ageing temperature, $Q_{10}$ value, desirable lifespan, and accelerated ageing time of the product.

3. The computer-implemented method of clause 1 wherein the historic ageing test data comprises the parameters, ageing temperature, $Q_{10}$ value, lifespan, and accelerated ageing time of the product.

4. The computer-implemented method of clause 1 wherein the at least one parameter of the product comprises factors relating to the environment, the polymer intrinsic properties of the product, and the structure of the product.

5. The computer-implemented method of clause 1 or 4 wherein the parameters relating to the environment comprise humidity, temperature, and pressure, and wherein these parameters are provided for the environments in which the product is assembled, intended to be stored, and intended to be used.

6. The computer-implemented method of clause 1 or 4 wherein the parameters relating to the structure of the product comprises the product user required specification, information about the interfaces between the plurality of materials in the product, and joining mechanisms between the plurality of materials in the product.

7. The computer-implemented method of any of clauses 1 to 6 wherein the step of obtaining weighted $Q_{10}$ values comprises the step of using a set of rules to add weighting to obtained $Q_{10}$ values of previous products with at least one parameter in common with the product.

8. The computer-implemented method of any of clauses 1 to 7 wherein the step of obtaining weighted $Q_{10}$ values of previous products with at least one parameter in common with the product using a set of rules comprises defining a weighting of the $Q_{10}$ values of previous products with at least one parameter in common with the product using the set of rules.

9. The computer-implemented method of any of clauses 1 to 8 wherein the set of rules are based on at least one of:

(i) historic ageing test data and failed test data;
(ii) the intended use of the product and the most prominent chemical or mechanical processes on the product; and
(iii) the duration and/or environmental conditions of different parts of the lifecycle of the product.

10. The computer-implemented method of any of clauses 1 to 9 wherein the outputted ageing test conditions and parameters of the product are added to the database for use in further identification of ageing test conditions for accelerated ageing tests of products.

11. A computer-implemented method for identifying the ageing test conditions for accelerated ageing tests of a product, wherein the method comprises:

obtaining parameters relating to the product;
determining the parameters of the product based on a lookup of previous products stored in a database;
identifying previous products with at least one parameter in common with the product;
determining a $Q_{10}$ value for the product based on the $Q_{10}$ value of a previous product in the database; and
outputting the $Q_{10}$ value for the product and an appropriate ageing temperature for use in accelerated ageing tests of the product.

12. The computer-implemented method of clause 11 wherein the parameters of the product comprises at least one of factors relating to the environment, the polymer intrinsic properties of the plurality of materials, and the structure of the product.

13. The computer-implemented method of clause 12 wherein the factors relating to the environment comprise humidity, temperature, and pressure, and wherein these parameters are provided for the environments in which the product is assembled, intended to be stored, and intended to be used.

14. The computer-implemented method of clause 12 wherein the structure of the product comprises the product user required specification, information about the interfaces between the plurality of materials, and the joining mechanisms between materials.

15. The computer-implemented method of clause 11 or 14 wherein the selective threshold is a 98.5% overlap of parameters in the product and previous products.

16. The computer-implemented method of any of clauses 11 to 15 wherein the product comprises only one material.

17. The computer-implemented method of clause 11 wherein the ageing test conditions is outputted for use in determining the lifespan of the product.

18. The computer-implemented method of any of clauses 11 to 17 wherein the ageing test conditions of the product are fed back to the database for use in further identifying the ageing test conditions for accelerated ageing tests of products.

19. A computer-implemented method for obtaining a $Q_{10}$ value for accelerated ageing tests of a product, wherein the method comprises:

performing a time temperature superposition technique on aged samples of the product;
obtaining material characteristic values for each of the aged samples of the product for each ageing duration;
iteratively determining a $Q_{10}$ value for the product that provides a closest coefficient of determination to 1;
outputting the determined $Q_{10}$ value for the product.

20. A computer-implemented method for obtaining a $Q_{10}$ value for accelerated ageing tests of a product, wherein the method comprises;

obtaining at least one $Q_{10}$ value of a first product;
performing differential scanning calorimetry on the first product;
determining the ratio of semicrystalline chain structure to amorphous chain structure present in the product, using the differential scanning calorimeter analysis data of the first product;
determining the ratio of semicrystalline chain structure to amorphous chain structure present in a second product using differential scanning calorimetry;
predicting the $Q_{10}$ value of a second material using (i) the $Q_{10}$ value of the first product, (ii) the determined ratio of semicrystalline chain structure to amorphous chain structure present in the first product, and (iii) the determined ratio of semicrystalline chain structure to amorphous chain structure present in the second product

**Claims**

1. A computer-implemented method for use in calculating the lifespan of a product, wherein the product comprises a plurality of materials, the method comprising:

obtaining parameters relating to the plurality of materials of the product;
obtaining a $Q_{10}$ value of each of the plurality of materials of the product, based on the obtained parameters;
determining an overall $Q_{10}$ value of the product, using a set of rules and the $Q_{10}$ values of the plurality of materials; and
outputting an appropriate ageing temperature and the overall $Q_{10}$ value for an accelerated ageing test of the product to calculate the lifespan of the product.

2. The computer-implemented method of claim 1 wherein the parameters relating to the plurality of materials comprises at least one of factors relating to the environment, the polymer intrinsic properties of the plurality of materials, and the structure of the product.

3. The computer-implemented method of claim 2 wherein the factors relating to the environment comprise humidity, temperature, and pressure, and wherein these parameters are provided for the environments in which the product is assembled, intended to be stored, and intended to be used.

4. The computer-implemented method of claim 2 wherein the structure of the product comprises the product user required specification, information about the interfaces between the plurality of materials, and the joining mechanisms between materials.

5. The computer-implemented method of any of claims 1 to 4 wherein the parameters relating to the plurality of materials are provided for the whole of the lifecycle of the product, and wherein determining an overall $Q_{10}$ value of the product comprises determining an overall $Q_{10}$ value of the product for the whole lifecycle of the product.

6. The computer-implemented method of any of claims 1 to 5 wherein the parameters of the plurality of materials are provided for different portions of the lifecycle of the product, wherein determining an overall $Q_{10}$ value of the product comprises determining an overall $Q_{10}$ value of the product for a selected portion of the lifecycle of the product, and wherein obtaining a $Q_{10}$ value of each of the plurality of materials of the product comprises performing a lookup in a database of $Q_{10}$ values.

7. The computer-implemented method of claim 6 wherein performing a lookup in a database of $Q_{10}$ values comprises selecting a $Q_{10}$ value of a material in the database that is similar to a material in the product, wherein the material is similar to a material in the database of $Q_{10}$ values if the parameters of the material are within a selected threshold level of similarity.

8. The computer-implemented method of any of the previous claims wherein obtaining parameters of the material of the product comprises performing a lookup in a database of parameters for materials in products, wherein the lookup is performed based on part names and classification and identifies a material that is similar to a material in the product, wherein the material is similar to a material in the database if the parameters of the material in the database and parameters of the material in the product are within a selected threshold level of similarity.

9. The computer-implemented method of claim 8 wherein the classifications include the intended use of the product, the factors relating to the environment, and the polymer intrinsic properties of the plurality of materials.

10. The computer-implemented method of any of the previous claims wherein materials of the product are similar to materials of a product in the database if the product is intended for the same use, if the materials have the same polymer intrinsic properties, or if the product faces the same environments when it is assembled, stored, and used.

11. The computer-implemented method of any of claims 1, 5, 6, or 7 wherein the $Q_{10}$ value of a material is obtained manually and the set of rules disregards any $Q_{10}$ values of 2.

12. The computer-implemented method of any of the previous claims wherein the step of determining an overall $Q_{10}$ value of the product, using a set of rules and the $Q_{10}$ values of the plurality of materials comprises selecting an overall $Q_{10}$ value of the product using the set of rules, from the plurality of $Q_{10}$ values of the materials of the product, as the highest $Q_{10}$ value if the spread of $Q_{10}$ values is less than a selected threshold.

13. The computer-implemented method of any of the previous claims wherein the step of determining an overall $Q_{10}$ value of the product, using a set of rules and the $Q_{10}$ values of the plurality of materials comprises selecting an overall $Q_{10}$ value of the product using the set of rules, from the plurality of $Q_{10}$ values of the materials of the product, as the median $Q_{10}$ value if the spread of $Q_{10}$ values is greater than a selected threshold, or defining a weighting of the Q10 values of each of the plurality of materials using the set of rules.

14. The computer-implemented method of any of the previous claims wherein the set of rules are based on at least one of:

   (i) historic ageing test data and failed test data;
   (ii) the intended use of the product and the most prominent chemical or mechanical processes on the product; and
   (iii) the duration and/or characteristics of different parts of the lifecycle of the product.

15. The computer-implemented method of claim 1 wherein the appropriate ageing temperature is obtained by the Arrhenius equation and the $Q_{10}$ value, ambient temperature, desirable lifespan and accelerated ageing time of the product, and wherein the outputted appropriate ageing temperature and the overall $Q_{10}$ value for an accelerated ageing test of the product are inputted into the memory of the database for use in further calculations of the lifespan of products.

Figure 1

200

Obtain parameters relating to the plurality of materials of the product (205)

Obtain the Q10 value of each of the plurality of materials of the product, based on the obtained parameters (210)

Determine an overall Q10 value of the product, using a set of rules and the Q10 values of the plurality of materials (215)

Output an appropriate ageing temperature and the overall Q10 value for an accelerated ageing test of the product to calculate the lifespan of the product (220)

Figure 2

300

Obtain parameters relating to the plurality of materials of the product (305)

Identify if the materials in a database comprise at least one of the obtained parameters, wherein the database comprises Q10 values of materials (310)

Identify if the parameters of the identified materials comprise a select threshold amount of the parameters of the plurality of materials of the product (315)

Obtain Q10 values of materials in the database which comprise at least a select threshold amount of the parameters of the plurality of materials of the product (315)

Obtain Q10 values manually for materials of the product that comprise less than a select threshold amount of the parameters of materials in the database (320)

Input the outputted appropriate ageing temperature and overall Q10 value for an accelerated ageing test of the product into the database for use in further product analysis (335)

Determine an overall Q10 value of the product, using a set of rules and the Q10 values of the plurality of materials (325)

Output an appropriate ageing temperature and the overall Q10 value for an accelerated ageing test of the product to calculate the lifespan of the product (330)

Figure 3

400

```
┌─────────────────────────────────────────────────────────────────┐
│  Store over 300 samples of the material in accelerated ageing    │
│  chambers of different temperatures for different durations (405) │
└─────────────────────────────────────────────────────────────────┘
                              │
                              ▼
┌─────────────────────────────────────────────────────────────────┐
│  Remove the samples and condition them at room temperature (RT)  │
│  for one to two hours (410)                                       │
└─────────────────────────────────────────────────────────────────┘
                              │
                              ▼
┌─────────────────────────────────────────────────────────────────┐
│  Load each sample incrementally and record the strain of the     │
│  material at each incremental load (415)                          │
└─────────────────────────────────────────────────────────────────┘
                              │
                              ▼
┌─────────────────────────────────────────────────────────────────┐
│  Obtain material characteristics for each aged sample (420)      │
└─────────────────────────────────────────────────────────────────┘
                              │
                              ▼
┌─────────────────────────────────────────────────────────────────┐
│  Calculate the real time equivalent for the accelerated ageing   │
│  duration of each sample using the Arrhenius equation (425)      │
└─────────────────────────────────────────────────────────────────┘
                              │
                              ▼
┌─────────────────────────────────────────────────────────────────┐
│  Plot the material characteristic against the logarithm of the   │
│  real time (430)                                                  │
└─────────────────────────────────────────────────────────────────┘
                              │
                              ▼
┌─────────────────────────────────────────────────────────────────┐
│  Alter the Q10 value in the real time calculation until the plot │
│  displays an R-squared value of approximately 1 and has linear   │
│  regression (435)                                                 │
└─────────────────────────────────────────────────────────────────┘
                              │
                              ▼
┌─────────────────────────────────────────────────────────────────┐
│  Output the Q10 value of the material (440)                      │
└─────────────────────────────────────────────────────────────────┘
```

Figure 4A

Figure 4B

500

```
                        ┌─────────────────────┐
                        │   Obtain a product  │
                        │        (505)        │
                        └─────────────────────┘
```

Material A (510) | Material B (525) | Material C (540) | Packaging (555)

Q10 of w (515) | Q10 of x (515) | Q10 of y (515) | Q10 of z (515)

Q10 of z (530)

Q10 of w (545) | Q10 of y (545) | Q10 of z (545)

Q10 of y (560) | Q10 of x (560) | Q10 of v (560)

Q10 of x (520)

Q10 of z (535)

Q10 of z (550)

Q10 of v (565)

Determining an overall Q10 value of the product using a set of rules (570)

Figure 5

600

Obtain a product
(605)

| Material A (610) | Material B (620) | Material C (630) | Material D (640) |
| --- | --- | --- | --- |
| Q10 of 2 (615) | Q10 of 3 (625) | Q10 of 6 (635) | Q10 of 11 (645) |

The values have large contrast so the overall determined Q10 value is the median, 4.5 (650)

Figure 6A

600'

```
              ┌─────────────────────┐
              │  Obtain a product   │
              │       (605')        │
              └─────────────────────┘
                         │
    ┌──────────┬─────────┼─────────┬──────────┐
    ▼          ▼                   ▼          ▼
┌─────────┐ ┌─────────┐      ┌─────────┐ ┌─────────┐
│Material A'│ │Material B'│      │Material C'│ │Material D'│
│ (610')  │ │ (620')  │      │ (630')  │ │ (640')  │
└─────────┘ └─────────┘      └─────────┘ └─────────┘
    │          │                   │          │
    ▼          ▼                   ▼          ▼
┌─────────┐ ┌─────────┐      ┌─────────┐ ┌─────────┐
│Q10 of 2 │ │Q10 of 2 │      │Q10 of 2 │ │Q10 of 15│
│ (615')  │ │ (625')  │      │ (635')  │ │ (645')  │
└─────────┘ └─────────┘      └─────────┘ └─────────┘
    └──────────┴─────────┬─────────┴──────────┘
                         ▼
        ┌────────────────────────────────────────┐
        │ The values have a conservative Q10 but the │
        │ contrast is high so the overall determined │
        │   Q10 value is the average 5.25 (650')   │
        └────────────────────────────────────────┘
```

Figure 6B

700

| Obtain at least one parameter of the product (705) |

| Perform a lookup in a database, wherein the database comprises historic ageing test data of previous products (710) |

| Identify at least one previous product which comprises at least one common parameter of the product (715) |

| Obtain weighted Q10 values of previous products with at least one parameter in common with the product using a set of rules (720) |

| Output the ageing test conditions of the previous product with the highest weighted Q10 value (725) |

Figure 7

800

Obtain at least one parameter of the product (805)

Perform a lookup in an internal database, wherein the database comprises historic ageing test data of previous products (810)

Internal database does not contain at least one previous product which comprises at least one common parameter of the product (820)

Internal database contains at least one previous product which comprises at least one common parameter of the product (815)

Perform a lookup in an external database, wherein the external database comprises historic ageing test data of previous products (825)

Input outputted ageing test conditions of the previous product with the highest weighted Q10 value of the product into the database for use in further product analysis (855)

External database contains at least one previous product which comprises at least one common parameter of the product (830)

Obtain weighted Q10 values of previous products with at least one parameter in common with the product using a set of rules (840)

Output the ageing test conditions of one previous product which has the highest risk Q10 value weighting (845)

Figure 8

900

Obtain parameters relating to the product (905)

Determine the parameters of the product based on a lookup of previous products stored in a database (910)

Identify previous products with at least one parameter in common with the product (915)

Determine a Q10 value for the product based on the Q10 value of a previous product in the database (920)

Output the Q10 value for the product and an appropriate ageing temperature for use in accelerated ageing tests of the product (925)

Figure 9

1000

Obtain parameters relating to the product (1005)

Determine the parameters of the product based on a lookup of previous products stored in an internal database (1010)

Input the Q10 value for the product and an appropriate ageing temperature for use in accelerated ageing tests of the product back into the internal database for use in further product analysis (1055)

Identify no previous products in the internal database comprising a select threshold amount of the parameters of the product (1025)

Identify at least one previous product in the internal database comprising a select threshold amount of the parameters of the product (1015)

Determine the parameters of the product based on a lookup of previous products stored in an external database (1030)

Identify at least one previous product in the external database comprising a select threshold amount of the parameters of the product (1035)

Identify no previous products in the external database comprising a select threshold amount of the parameters of the product (1040)

Determine a Q10 value for the product based on the Q10 value of a previous product in the database (1020)

Obtain a Q10 value for the product manually (1045)

Output the Q10 value for the product and an appropriate ageing temperature for use in accelerated ageing tests of the product (1050)

Figure 10

Figure 11

Figure 12

Accumulated life span, 4 years 2 months

1 month under load, at 20° (120)

3 years in assembly condition, at 20° and in humidity (115)

1 month being assembled at 40° (110)

1 year as constituent parts under no load, at 20° (105)

Years

Figure 13

Figure 14A

Figure 14B

Figure 15A

Figure 15B

## EP 4 621 674 A1

**EUROPEAN SEARCH REPORT**

Europäisches Patentamt
European Patent Office
Office européen des brevets

Application Number

EP 25 15 9512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Petrie Glenn: "The Need for Specificity in Accelerated Aging", Medical Device and Diagnostic Industry, 1 October 2006 (2006-10-01), pages 1-5, XP093297698, Retrieved from the Internet: URL:https://www.mddionline.com/digital-health/the-need-for-specificity-in-accelerated-aging [retrieved on 2025-06-23] * the whole document * | 1-15 | INV. G06Q10/04 G01N33/00 G06F30/20 G06Q10/30 G06Q50/04 G16C60/00 |
| A | Astm: "F1980-02: Accelerated Aging of Sterile Medical Device Packages", , 10 January 2002 (2002-01-10), pages 1-6, XP093301245, Retrieved from the Internet: URL:https://file.yizimg.com/424653/2013120515134271.pdf [retrieved on 2025-07-31] * the whole document * | 1-15 | |

TECHNICAL FIELDS SEARCHED (IPC)

G06Q
G01N
G06F
G16C

The present search report has been drawn up for all claims

| Place of search | Date of completion of the search | Examiner |
|---|---|---|
| Munich | 31 July 2025 | Härdeman, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)